# EUROPEAN PATENT APPLICATION

(11) **EP 2 514 406 A1**
(43) Date of publication of application: **24.10.2012**
(21) Application number: 12159416.2
(22) Date of filing: 30.05.2008
(51) Int. Cl.: A61K 9/00, A61K 9/08, A61K 9/19, A61K 38/04, A61K 38/16, A61K 47/10, A61K 47/42, A61K 9/12, A61K 38/28

(54) **Spontaneously dispersible preconcentrates including a peptide drug in a solid or semisolid carrier**

(30) Priority: 01.06.2007 EP 07109435; 17.08.2007 EP 07114524; 15.11.2007 EP 07120807
(62) Divisional of application: 08760275.1
(71) Applicant: Novo Nordisk A/S, 2880 Bagsværd (DK)
(72) Inventor: Bjerregaard Jensen, Simon, 2880 Bagsvaerd (DK); Havelund, Svend, 2880 Bagsvaerd (DK); Föger, Florian Anders, 2880 Bagsvaerd (DK)

(57) **Abstract**

The present invention relates to a solid or semisolid composition comprising a polypeptide (a), at least one polar organic solvent (b) for the polypeptide, at least one surfactant (c), at least one lipophilic component (d), and optionally at least one solid hydrophilic component (e), wherein said pharmaceutical composition is spontaneously dispersible, is provided.

## Description

### FIELD OF THE INVENTION

The present invention relates to a pharmaceutical composition, e.g. a microemulsion preconcentrate that includes a peptide drug in a solid or semisolid carrier.

### BACKGROUND OF THE INVENTION

The oral route is by far the most widely used route for drug administration and is in general very well accepted by patients, especially for chronic therapies. Administration of therapeutic peptides or proteins is however often limited to parenteral routes rather than the preferred oral administration due to several barriers such as enzymatic degradation in the gastrointestinal (GI) tract, drug efflux pumps, insufficient and variable absorption from the intestinal mucosa, as well as first pass metabolism in the liver. Human insulin is degraded by various digestive enzymes found in the stomach (pepsin), in the intestinal lumen (chymotrypsin, trypsin, elastase, carboxypeptidases, etc.) and in mucosal surfaces of the GI tract (aminopeptidases, carboxypeptidases, enteropeptidases, dipeptidyl peptidases, endopeptidases, etc.).

This is unfortunate because many peptides and many proteins have proven clinically effective and could have more widespread use if easy to administer and acceptable to recipients.

Diabetes mellitus is a metabolic disorder in which the ability to utilize glucose is partly or completely lost which may be treated with e.g. insulin.

The general approach for peptide and protein delivery such as insulin delivery is parenteral administration which is invasive and inconvenient. Therefore non-invasive routes like oral delivery of protein based pharmaceuticals are increasingly investigated. Recent formulation designs for oral protein/peptide delivery include co-formulations with protease inhibitors, permeation enhancers, polymer-based delivery systems and insulin conjugates.

A particularly useful vehicle for oral administration of a drug to a mammal, e.g., a human, is in the form of a microemulsion preconcentrate. A microemulsion preconcentrate, e.g., includes at least oneoil or other lipophilic ingredients, at least one surfactant, optional hydrophilic ingredients, and any other agents or excipients as needed. When the components of the system contact anaqueous medium, e.g., water, a microemulsion spontaneously forms, such as an oil-in-watermicroemulsion, with little or no agitation. The resulting microemulsion is a thermodynamically stable system comprising two immiscible liquids, in which one liquid isfinely divided into the other because of the presence of a surfactant(s). The microemulsionformed, e.g.,appears clear or translucent, slightly opaque, opalescent, non-opaque or substantially non-opaque because of the low particle size of the dispersed phase.

It has now been surprisingly found that particularly suitable compositions for oral administration containing therapeutically active water soluble polypeptides such as insulin having particularly interesting bioavailability characteristics, improved stability and improvedprocessingsuch asease of filling into pharmaceutically acceptable capsulesare obtainable using a pharmaceutical composition according to the invention.

### SUMMARY OF THE INVENTION

The present invention relates to a pharmaceutical composition, e.g. a microemulsion preconcentrate that includes a peptide drug in a solid or semisolid carrier. The system forms an emulsion, e.g., a microemulsion when brought in contact with an aqueous medium, e.g., water or the gastric juices of the gastrointestinal tract.

In one aspect of the invention, a solid or semi-solid pharmaceutical composition comprising a polypeptide (a), at least one polar organic solvent (b) for the polypeptide, at least one surfactant (c), at least one lipophilic component (d), and optionally at least one solid hydrophilic component (e), wherein said pharmaceutical composition is spontaneously dispersible, is provided.

In a further aspect of the invention, a solid or semi-solid pharmaceutical composition comprising a water soluble polypeptide (a), at least one polar organic solvent (b) for the polypeptide, at least one surfactant (c), at least one lipophilic component (d), and optionally at least onesolid hydrophilic component (e), wherein said pharmaceutical composition is spontaneously dispersible, is provided.

### DESCRIPTION OF THE DRAWINGS

**Figure 1**: Reduction in blood glucose levels in non diabetic dogs by oral administration of a pharmaceutical composition according to the invention comprising insulin aspart.
**Figure 2**: Reduction in blood glucose levels in non diabetic dogs by oral administration of a pharmaceutical composition according to the invention comprising an insulin analog.
**Figure 3**: Reduction in blood glucose levels in non diabetic rats by oral administration of a pharmaceutical composition according to the invention comprising insulin aspart.
**Figure 4**: Reduction in blood glucose levels in non diabetic rats by oral administration of a pharmaceutical composition according to the invention comprising an insulin analog.

### DESCRIPTION OF THE INVENTION

The invention relates to a solid or semi-solid pharmaceutical composition comprising a polypeptide (a), at least one polar organic solvent (b) for the polypeptide, at least one surfactant (c), at least one lipophilic component (d), and optionally at least one solid hydrophilic component (e), wherein said pharmaceutical composition is spontaneously dispersible.

In one aspect, the invention provides a solid or semi-solid pharmaceutical composition comprising a water soluble polypeptide (a), a polar organic solvent (b) for the polypeptide, a surfactant (c), a lipophilic component (d), and optionally a solid hydrophilic component (e), wherein said pharmaceutical composition is spontaneously dispersible.

It has surprisingly been found that particularly suitable solid or semi-solid compositions for oral administration comprising therapeutically active water soluble polypeptides, such as insulin, and hydrophilic component(s) are obtainable using a pharmaceutical composition according to the invention.

The composition according to the invention has thus surprisingly been found to enhance the efficacy of uptake of peptides administered orally.

Also, the polypeptides in the composition according to the inventionhave been found tohave improved stability relative to the free polypeptides, i.e. polypeptides thatare not formulated in the composition.

The present invention relates to a pharmaceutical composition, i.e., a spontaneously dispersiblepreconcentrate that includes a therapeutically active water-soluble polypeptide in a carrier that comprisesa lipophilic component, a surfactant and a polar organic solvent and optionally a solid hydrophilic component (e). In the aspect where there is no solid hydrophilic component present at least one of the components selected from the group consisting of a lipophilic component and a surfactant is solid or semi-solid. In the aspect where there is a solid hydrophilic component (e) present both the lipophilic component and the surfactant may be liquid. In one aspect, the surfactant is solid. In one aspect, a solid hydrophilic component is present.

As used herein, the term "carrier" refers to the pharmaceutically acceptable vehicle that transports the therapeutically active water-soluble polypeptideacross the biological membrane or within a biological fluid. The carrier, of the present invention, comprisesa lipophilic component, a polar organic solvent and a surfactant, and optionally a solid hydrophilic component. The carrier of the present invention is capable of spontaneously producing a microemulsion or colloidal structures, when brought in contact, dispersed, or diluted, with an aqueous medium, e.g., water, fluids containing water, or in *vivo* media in mammals, such as the gastric juices of the gastrointestinal tract. The colloidal structures can be solid or liquid particles including domains, droplets, micelles and nanoparticles.

When the pharmaceutical composition is brought into contact with an aqueous medium, an emulsion, especially a microemulsion, spontaneously forms. In particular, an emulsion or microemulsion forms in the digestive tract of a mammal when the delivery system of the present invention is orally ingested. In addition to the aforementioned components, the spontaneously dispersiblepreconcentrate can also optionally contain other excipients, such as buffers, pH adjusters, stabilizers and other adjuvants recognized by one of ordinary skill in the art to be appropriate for such a pharmaceutical use.

In one aspect of the invention, the pharmaceutical composition is non-aqueous. The term "non-aqueous" as used herein refers to a composition which comprises less than 20% w/w water. In a more preferred embodiment, the composition according to the invention comprises less than 10% w/w water. In a more preferred embodiment, the composition according to the invention comprises less than 8% w/w water, in a more preferred embodiment less than 5% w/w water, in a more preferred embodiment less than 3% w/w water and in an even more preferred embodiment less than 2% w/w water.

As used herein, the term "microemulsion preconcentrate" means a composition, which spontaneously forms a microemulsion, e.g., an oil-in-water microemulsion, in an aqueous medium, e.g. in water or in the gastrointestinal fluids after oral application.The composition self-emulsifies upon dilution in an aqueous medium for example in a dilution of 1:5, 1:10, 1:50, 1:100 or higher. Thespontaneously dispersiblepreconcentrate according to the invention comprises a lipophilic component, a surfactant, and an organic polar component. The polar component and the surfactant togetherin the drug delivery system can comprise up to 95% by weight of the composition of the carrier, e.g., 80%.In a further aspect, the polar component and the surfactant togetherin the drug delivery system comprises from 5% to 90% by weight of the composition of thecarrier.In yet a further aspect, the polar component and the surfactant togetherin the drug delivery system comprisesfrom 20% to 50% by weight of the composition of the carrier.

The spontaneously dispersiblepreconcentrate may be solid or semi-solid. As used herein, the term "solid" means a component or composition that is in a solidstate at room temperature ("RT"), and having a melting point of, for example, above 40°C, e.g., up to about 65°C. As used herein room temperature (RT) means approximately 20-25°C.

As used herein, the term "semi-solid" relates to a component or composition whichis not liquid at room temperature, e.g., having a melting pointbetween room temperature and about 40°C. A semisolid can have the qualities and/or attributes of both the solid and liquid states of matter. As used-herein, the term "solidify" means to make solid or semi-solid.

As used herein, the term "microemulsion" refers to a clear or translucent, slightly opaque, opalescent, non-opaque or substantially non-opaque colloidal dispersion that is formed spontaneously or substantially spontaneously when its components are brought into contact with an aqueous medium.

A microemulsion is thermodynamically stable and contains homogenously dispersed particles or domains, for example of a solid or liquid state (e.g., liquid lipid particles or droplets), of a mean diameter less than about 500 nm, e.g., less than about 400 nm or less than 300 nm, less than 200 nm, less than 100 nm, and greater than about 2-4 nm as measured by standard light scattering techniques, e.g., using a MALVERN ZETASIZER Nano ZS. Solid particles in a microemulsion can be amorphous or crystalline in nature which can, for example, have particle sizes greater than 300 nm. Microemulsions, e.g., are thermodynamically stable, e.g., for at least fifteen minutes, or up to four hours or even twenty-four hours or longer. The term "domain size" as used herein refers to repetitive scattering units and can be measured by e.g., small angle X-ray. In one aspect of the invention, the domain size is smaller than 400 nm, more preferred smaller than 300 nm and most preferred smaller than 200 nm.

As used herein the term "spontaneously dispersible" when referring to a pre-concentrate refers to acomposition that is capable of producing colloidal structures such as microemulsions, emulsions and other colloidal systems, when diluted with an aqueous medium when the components of the composition of the invention are brought into contact with an aqueous medium, e.g. by simple shaking by hand for a short period of time, for example for ten seconds. In one aspect a spontaneously dispersible concentrate according to the invention is a microemulsion pre-concentrate.

Microemulsions can offer greater ease of preparation due to spontaneous formation, thermodynamic stability and elegant aesthetics. Microemulsions improve the delivery of the polypeptide because they can increase drug loading, enhance penetration, reduce particle size, improve particle size uniformity, increase dissolution rate, increase bioavailability and reduce inter- and intra-individual variability in drug pharmacokinetics as compared to traditional coarse emulsions.

As used herein, the term "lipophilic component" refers to a substance, material oringredient that is more compatible with oil than with water. A material with lipophilicproperties is insoluble or almost insoluble in water but is easily soluble in oil or othernonpolar solvents. The term "lipophilic component" can comprise one or more lipophilicsubstances. Multiple lipophilic components may constitute the lipophilic phase of the spontaneously dispersiblepreconcentrate and form the oil aspect, e.g.,in an oil-in-water microemulsion. Atroom temperature, the lipophilic component and lipophilic phase of the spontaneously dispersiblepreconcentrate can be solid, semisolid or liquid. For example, a solid lipophilic componentcan exist as a paste, granular form, powder or flake. If more than one excipient comprises the lipophilic component, the lipophilic component can be a mixture of liquids, solids, or both.

For example, the lipophilic component comprises from about 5% to about 85 % by weight of the composition, e.g., from about 10% to about 85%, e.g., from about 15% to about 60%, e.g., from about 20% to about 40%.

Examples of solid lipophilic components, i.e., lipophilic components which are solid or semisolid at room temperature,include, but are not limited to, the following:
1. mixtures of mono-, di- and triglycerides, such as hydrogenated coco-glycerides(melting point (m.p.) of about 33.5°C to about 37°C], commercially-available asWITEPSOL HI5from Sasol Germany (Witten, Germany); Examples of fatty acid triglycerides e.g., C₁₀-C₂₂ fatty acid triglycerides include natural and hydrogenated oils,such as vegetable oils;
2. esters, such as propylene glycol (PG) stearate, commercially available as MONOSTEOL (m.p. of about 33°C to about 36°C) from Gattefosse Corp. (Paramus,NJ); diethylene glycol palmito stearate, commercially available as HYDRINE (m.p. ofabout 44.5°C to about 48.5°C) from Gattefosse Corp.;
3. polyglycosylated saturated glycerides, such as hydrogenated palm/palm kerneloil PEG-6esters (m.p. of about 30.5°C to about 38°C), commercially-available asLABRAFIL M2130 CS from Gattefosse Corp. or Gelucire 33/01;
4. fatty alcohols, such as myristyl alcohol (m.p. of about 39°C), commerciallyavailableas LANETTE 14 from Cognis Corp. (Cincinnati, OH); esters of fatty acids with fatty alcohols, e.g., cetyl palmitate (m.p. of about 50°C); isosorbid monolaurate,e.g. commercially available under the trade name ARLAMOLISML from Uniqema(New Castle, Delaware), e.g. having a melting point of about 43°C;
5. PEG-fatty alcohol ether, including polyoxyethylene (2) cetyl ether, e.g.commercially available as BRIJ 52 from Uniqema, having a melting point of about 33°C, or polyoxyethylene (2) stearyl ether, e.g. commercially available as BRIJ 72 from Uniqema having a melting point of about 43°C;
6. sorbitan esters, e.g. sorbitan fatty acid esters, e.g. sorbitan monopalmitate orsorbitan monostearate, e.g,commercially available as SPAN 40 or SPAN 60 fromUniqema and having melting points of about 43°C to 48°C or about 53°C to 57°C and 41°C to 54°C, respectively; and
7. glyceryl mono-C₆-C₁₄-fatty acid esters. These are obtained by esterifying glycerolwith vegetable oil followed by molecular distillation. Monoglycerides include, but are not limited to, both symmetric (i.e. β-monoglycerides) as well as asymmetric monoglycerides (α-monoglycerides). They also include both uniform glycerides (in which the fatty acid constituent is composed primarily of a single fatty acid) as well asmixed glycerides (i.e. in which the fatty acid constituent is composed ofvarious fattyacids).Thefatty acid constituent may include both saturated and unsaturated fattyacids having a chain length of from e.g. C₈-C₁₄. Particularly suitable are glycerylmono laurate e.g. commercially available as IMWITOR 312 from Sasol NorthAmerica (Houston, TX), (m.p. of about 56°C-60°C); glyceryl mono dicocoate, commercially available as IMWITOR 928 from Sasol (m.p. of about 33°C - 37°C); monoglyceryl citrate, commercially available as IMWITOR 370, (m.p. of about 59 toabout 63°C); or glyceryl mono stearate, e.g.,commercially available as IMWITOR900 from Sasol (rn.p. of about 56°C-61°C); or self-emulsifying glycerol mono stearate, e.g., commercially available as IMWITOR 960 from Sasol (m.p.of about 56°C-61°C).

Examples of liquid lipophilic components, i.e., lipophilic components which are liquid at room temperature include, butare not limited to, the following:
1. mixtures of mono-, di- and triglycerides, such as medium chain mono- and diglycerides,glyceryl caprylate/caprate, commercially-available as CAPMUL MCMfrom Abitec Corp. (Columbus, OH);
2. glyceryl mono- or di fatty acid ester, e.g. of C₆-C₁₈, e.g. C₆-C₁₆ e.g. C₈-C₁₀, e.g. C₈, fatty acids, or acetylated derivatives thereof, e.g. MYVACET 9-45 or 9-08 fromEastman Chemicals (Kingsport, TN) or IMWITOR 308 or 312 from Sasol;
3. propylene glycol mono- or di- fatty acid ester, e.g. of C₈-C₂₀, e.g. C₈-C₁₂, fattyacids, e.g. LAUROGLYCOL 90, SEFSOL 218, or CAPRYOL 90 or CAPMUL PG-8 from Abitec Corp.;
4.oils, such as safflower oil, sesame oil, almond oil, peanut oil, palm oil, wheatgerm oil, corn oil, castor oil, coconut oil, cotton seed oil, soybean oil, olive oil and mineral oil;
5. fatty acids or alcohols, e.g. C₈-C₂₀, saturated or mono-or di- unsaturated, e.g.oleic acid, oleyl alcohol, linoleic acid, capric acid, caprylic acid, caproic acid,tetradecanol, dodecanol, decanol;
6. medium chain fatty acid triglycerides, e.g. C₈-C₁₂, e.g. MIGLYOL 812, or longchain fatty acid triglycerides, e.g. vegetable oils;
7. transesterified ethoxylated vegetable oils, e.g. commercially available asLABRAFIL M2125 CS from Gattefosse Corp;
8. esterified compounds of fatty acid and primary alcohol, e.g. C₈-C₂₀, fatty acids and C₂-C₃ alcohols, e.g. ethyl linoleate, e.g. commercially available as NIKKOL VF-E fromNikko Chemicals (Tokyo, Japan), ethyl butyrate, ethyl caprylateoleic acid, ethyloleate, isopropyl myristate and ethyl caprylate;
9. essential oils, or any of a class of volatile oils that give plants their characteristicodors, such as spearmint oil, clove oil, lemon oil and peppermint oil;
10. fractions or constituents of essential oils, such as menthol, carvacrol and thymol;
11. synthetic oils, such as triacetin, tributyrin;
12. triethyl citrate, acetyl triethyl citrate, tributyl citrate, acetyl tributyl citrate;
13. polyglycerol fatty acid esters, e.g. diglyceryl monooleate, e.g. DGMO-C, DGMO-90, DGDO from Nikko Chemicals; and
14. sorbitan esters, e.g. sorbitan fatty acid esters, e.g. sorbitanmonolaurate, e.g.commercially available as SPAN 20 from Uniqema.
15. Phospholipids, e.g.Alkyl-O-Phospholipids, Diacyl Phosphatidic Acids, Diacyl Phosphatidyl Cholines, Diacyl Phosphatidyl Ethanolamines, Diacyl Phosphatidyl Glycerols, Di-O-Alkyl Phosphatidic Acids, L-alpha-Lysophosphatidylcholines (LPC), L-alpha-Lysophosphatidylethanolamines (LPE), L-alpha-Lysophosphatidylglycerol (LPG), L-alpha-Lysophosphatidylinositols (LPI) L-alpha-Phosphatidic acids (PA), L-alpha-Phosphatidylcholines (PC), L-alpha-Phosphatidylethanolamines (PE), L-alpha-Phosphatidylglycerols (PG), Cardiolipin (CL), L-alpha-Phosphatidylinositols (PI), L-alpha-Phosphatidylserines (PS), LysoPhosphatidylcholines, Lyso-Phosphatidylglycerols, sn-Glycerophosphorylcholines commercially available from LARODAN, or soybean phospholipid (Lipoid S100) commercially available from Lipoid GmbH.

In one aspect of the invention, the lipophilic component is one or more selected from the group consisting of mono-, di-, and triglycerides. In a further aspect, the lipophilic component is one or more selected from the group consisting of mono- and diglycerides. In yet a further aspect, the lipophilic component is Capmul MCM.

The term "polar solvent" refers in one aspect herein to a "polar protic organic solvent" which is a hydrophilic, water miscible carbon-containing solvent that contains an O-H or N-H bond, or mixtures thereof.The polarity is reflected in the dielectric constant or the dipole moment of a solvent. The polarity of a solvent determines what type of compounds it is able to dissolve and with what other solvents or liquid compounds it is miscible. Typically, polar solvents dissolve polar compounds best and non-polar solvents dissolve non-polar compounds best: "like dissolves like". Strongly polar compounds like inorganic salts (e.g. sodium chloride) dissolve only in very polar solvents.

In a further aspect of the invention, the polar solvent is a solvent having a dielectricity constant above 20, preferably in the range of 20-50. Examples of different polar solvents are listed in Table 1 together with water as a reference.

**Table 1. Dielectricity constants (static permittivity) of selected polar organic protic solvents and water as a reference (Handbook of Chemistry and Physics, CMC Press, dielectricity constants are measured in static electric fields or at relatively low frequencies, where no relaxation occurs)**

| Solvent (Temperature , Kelvin) | Dielectricity constant, ε* |
|---|---|
| Water (293.2) | 80.1 |
| Propanetriol [Glycerol] (293.2) | 46.53 |
| Ethanediol [Ethylene Glycol] (293.2) | 41.4 |
| 1,3-propanediol (293.2) | 35.1 |
| Methanol (293.2) | 33.0 |
| 1,4-butanediol (293.2) | 31.9 |
| 1,3-butanediol (293.2) | 28.8 |
| 1,2-propanediol [propylene glycol] (303.2) | 27.5 |
| Ethanol (293.2) | 25.3 |
| Isopropanol (293.2) | 20.18 |

In the present context, 1,2-propanediol and propylene glycol is used interchangeably. In the present context, propanetriol and glycerol is used interchangeably. In the present context, ethanediol and ethylene glycol is used interchangeably.

In one aspect of the invention, the solvent is selected from the group consisting of polyols. The term "polyol" as used herein refers to chemical compounds containing multiple hydroxyl groups.

In a further aspect of the invention, the solvent is selected from the group consisting of diols and triols. The term "diol" as used herein refers to chemical compounds containing two hydroxyl groups. The term "triol" as used herein refers to chemical compounds containing three hydroxyl groups.

In a further aspect of the invention, the solvent is selected from the group consisting of glycerol (propanetriol), ethanediol (ethylene glycol), 1,3-propanediol, methanol, 1,4-butanediol, 1,3-butanediol, propylene glycol (1,2-propanediol), ethanol and isopropanol, or mixtures thereof. In a further aspect of the invention, the solvent is selected from the group consisting of propylene glycol and glycerol. In a preferred aspect of the invention, the solvent is glycerol. This solvent is biocompatible even at high dosages and has a high solvent capacity for e.g. insulin peptides and GLP-1 compounds. In another preferred aspect of the invention, the solvent is selected from the group consisting of propylene glycol and ethylene glycol. These solvents have a low viscosity, are biocompatible at moderate doses, and have very high solvent capacity for e.g. insulin peptides and GLP-1 compounds.

The solvents should preferably be of high purity with a low content of e.g. aldehydes, ketones and other reducing impurities in order to minimize chemical deterioration of the solubilized polypeptide due to e.g. Maillard reaction. Scavenger molecules like glycylglycine and ethylenediamine may be added to the formulations comprising semi-polar protic organic solvent(s) such as polyols to reduce deterioration of the polypeptide whereas antioxidants can be added to reduce the rate of formation of further reducing impurities.

In one aspect of the invention, the organic polar solvent is present in the pharmaceutical composition in an amount of 5-80% w/w. In a further aspect of the invention, the organic polar solvent is present in an amount of 10-70% w/w. In a further aspect of the invention, the organic polar solvent is present in an amount of 20-60% w/w. In a further aspect of the invention, the organic polar solvent is present in an amount of 30-50% w/w. In a further aspect of the invention, the organic polar solvent is present in an amount of 35-45% w/w.

In one aspect of the invention, the organic polar solvent is selected from the group consisting of glycerol, propylene glycol and mixtures thereof. In a further aspect, the organic polar solvent is glycerol. In a further aspect, the organic polar solvent is a mixture of glycerol and propylene glycol. In yet a further aspect, the organic polar solvent is propylene glycol.

A solid hydrophilic component may be added to the spontaneously dispersible preconcentratein order to render or help render the spontaneously dispersible preconcentrate solid or semi-solid atroom temperature. The hydrophilic component can comprise more than one excipient. Ifmore than one excipient comprises the hydrophilic component, the hydrophilic componentcan be a mixture of liquids, solids, or both.

The hydrophilic component, when present, may comprise from about 0% to about 70%by weight of the composition, e.g., from about 10% to about 50%,e.g., from about 10% toabout 40%, e.g. from about 10% to about 30%.

An example of a hydrophilic component is PEG which is the polymer of ethyleneoxide that conforms generally to the formula H(OCH₂CH₂)ₙ0H in which n correlates with the average molecular weight of the polymer.

The types of PEG useful in the present invention can be categorized by its state ofmatter, i.e., whether the substance exists in a solid or liquid form at room temperature and pressure. As used herein, "solid PEG" refers to PEGhaving a molecular weight such that the substance is in a solid state at room temperature and pressure. For example, PEG having a molecular weight ranging between 1,000 and 10,000 is a solid PEG. Such PEGsinclude, but are not limited to PEG 1000, PEG 1550, PEG 2000, PEG 3000, PEG 3350, PEG4000 or PEG 8000. Particularly useful solid PEGs are those having a molecular weightbetween 1,450 and 8,000. Especially useful as a solid PEG are PEG 1450, PEG 3350, PEG4000, PEG 8000, derivatives thereof and mixtures thereof. PEGs of various molecularweights are commercially-available as the CARBOWAX SENTRY series from DowChemicals (Danbury, CT). Moreover, solid PEGs have a crystalline structure, or polymericmatrix, which is a particularly useful attribute in the present invention, Polyethylene oxide ("PEO") which has an identical structure to PEG but for chain length and end groups are alsosuitable for use in the present invention. Various grades of PEO are commercially availableas POLYOX from Dow Chemicals. PEO, for example, has a molecular weight ranging fromabout 100,000 to 7,000,000. The hydrophilic component in the present invention cancomprise PEG, PEO, and any combinations of the foregoing.

The hydrophilic components of the present invention can optionally include a loweralkanol, e.g.,ethanol. While the use of ethanol isnot essential, it canimprove solubility of the polypeptide in the carrier, improve storage characteristics and/or reduce the risk of drug precipitation.

In an alternative exemplary embodiment, the hydrophilic component of the carrierconsists of a single hydrophilic component, e.g., a solid PEG, e.g., PEG 1450, PEG 3350,PEG 4000 and PEG 8000. In this exemplary embodiment, the hydrophilic phase of the microemulsion component consists of a single hydrophilic substance. For example, if the carrier comprised PEG 3350, the carrier would contain no other hydrophilic substances, e.g., lower alkanols (lower alkyl being C₁-C₄), such as ethanol; or water.

In yet another alternative exemplary embodiment, the hydrophilic component of the carrier consists of a mixture of solid PEGs. For example, the hydrophilic componentcomprises PEG 1450, PEG 3350, PEG 4000, PEG 8000, derivatives thereof and anycombinations and mixtures thereof.

The carrier also comprises one or more surfactants, i.e., optionally a mixture of surfactants; orsurface active agents, which reduce interfacial tension. The surfactant is e.g., nonionic,ionic or amphoteric. Surfactants can be complex mixtures containing side products orun-reacted starting products involved in the preparation thereof, e.g., surfactants made bypolyoxyethylation may contain another side product, e.g., PEG.The surfactant orsurfactants can have any HLB that is useful in the pharmaceutical arts. For example, the surfactant may have a hydrophilic-lipophilic balance (HLB) having a mean HLB value of 8-30, e.g.,15-30. The surfactants can also be liquid or solid in nature.

The term "surfactant" as used herein refers to any substance, in particular a detergent, that can adsorb at surfaces and interfaces, like liquid to air, liquid to liquid, liquid to container or liquid to any solid. The surfactant may be selected from a detergent, such as ethoxylated castor oil, polyglycolyzed glycerides, acetylated monoglycerides, sorbitan fatty acid esters, polysorbate, such as polysorbate-20, poloxamers, such as poloxamer 188 and poloxamer 407, polyoxyethylene sorbitan fatty acid esters, polyoxyethylene derivatives such as alkylated and alkoxylated derivatives (tweens, e.g. Tween-20, or Tween-80), monoglycerides or ethoxylated derivatives thereof, diglycerides or polyoxyethylene derivatives thereof, glycerol, cholic acid or derivatives thereof, lecithins, alcohols and phospholipids, glycerophospholipids (lecithins, cephalins, phosphatidyl serine), glyceroglycolipids (galactopyransoide), sphingophospholipids (sphingomyelin), and sphingoglycolipids (ceramides, gangliosides), DSS (docusate sodium, CAS registry no [577-11-7]), docusate calcium, CAS registry no [128-49-4]), docusate potassium, CAS registry no [7491-09-0]), SDS (sodium dodecyl sulfate or sodium lauryl sulfate), dipalmitoyl phosphatidic acid, sodium caprylate, bile acids and salts thereof and glycine or taurine conjugates, ursodeoxycholic acid, sodium cholate, sodium deoxycholate, sodium taurocholate, sodium glycocholate, N-hexadecyl-N,N-dimethyl-3-ammonio-1-propanesulfonate, anionic (alkyl-aryl-sulphonates) monovalent surfactants, palmitoyl lysophosphatidyl-L-serine, lysophospholipids (e.g. 1-acyl-sn-glycero-3-phosphate esters of ethanolamine, choline, serine or threonine), alkyl, alkoxyl (alkyl ester), alkoxy (alkyl ether)- derivatives of lysophosphatidyl and phosphatidylcholines, e.g. lauroyl and myristoyl derivatives of lysophosphatidylcholine, dipalmitoylphosphatidylcholine, and modifications of the polar head group, that is cholines, ethanolamines, phosphatidic acid, serines, threonines, glycerol, inositol, and the postively charged DODAC, DOTMA, DCP, BISHOP, lysophosphatidylserine and lysophosphatidylthreonine, zwitterionic surfactants (e.g. N-alkyl-N,N-dimethylammonio-1-propanesulfonates, 3-cholamido-1-propyldimethylammonio-1-propanesulfonate, dodecylphosphocholine, myristoyl lysophosphatidylcholine, hen egg lysolecithin), cationic surfactants (quaternary ammonium bases) (e.g. cetyltrimethylammonium bromide, cetylpyridinium chloride), non-ionic surfactants (e. g. alkyl glucosides like dodecyl β-D-glucopyranoside, dodecyl β-D-maltoside, tetradecyl β-D-glucopyranoside, decyl β-D-maltoside, dodecyl β-D-maltoside, tetradecyl β-D-maltoside, hexadecyl β-D-maltoside, decyl β-D-maltotrioside, dodecyl β-D-maltotrioside, tetradecyl β-D-maltotrioside, hexadecyl β-D-maltotrioside, n-dodecyl-sucrose, n-decyl-sucrose, fatty alcohol ethoxylates (e. g. polyoxyethylene alkyl ethers like octaethylene glycol mono tridecyl ether, octaethylene glycol mono dodecyl ether, octaethylene glycol mono tetradecyl ether), block copolymers as polyethyleneoxide/polypropyleneoxide block copolymers (Pluronics/Tetronics, Triton X-100) ethoxylated sorbitan alkanoates surfactants (e. g. Tween-40, Tween-80, Brij-35), fusidic acid derivatives (e.g. sodium tauro-dihydrofusidate etc.), long-chain fatty acids and salts thereof C8-C20(eg. oleic acid and caprylic acid), acylcarnitines and derivatives, N^{α}-acylated derivatives of lysine, arginine or histidine, or side-chain acylated derivatives of lysine or arginine, N^{α}-acylated derivatives of dipeptides comprising any combination of lysine, arginine or histidine and a neutral or acidic amino acid, N^{α}-acylated derivative of a tripeptide comprising any combination of a neutral amino acid and two charged amino acids, or the surfactant may be selected from the group of imidazoline derivatives, or mixtures thereof.

### Examples of solid surfactants include, but are not limited to,

1. reaction products of a natural or hydrogenated castor oil and ethylene oxide. Thenatural or hydrogenated castor oil may be reacted with ethylene oxide in a molar ratioof from about 1:35 to about 1:60, with optional removal of the PEG component from the products. Various such surfactants are commerciallyavailable, e-g., theCREMOPHOR series from BASF Corp. (Mt. Olive, NJ), such as CREMOPHORRH 40 which is PEG40 hydrogenated castor oil which has a saponification value ofabout 50- to 60, an acid value less than about one, a water content, i.e., Fischer, lessthan about 2%, an n_{D}⁶⁰ of about 1.453-1.457, and an HLB of about 14-16;
2. polyoxyethylene fatty acid esters that include polyoxyethylene stearic acid esters,such as the MYRJ series from Uniqema e.g., MYRJ 53 having a m.p. of about 47°C. Particular compounds in the MYRJ series are, e.g., MYRJ 53 having a m.p. of about 47°C and PEG-40-stearate available as MYRJ 52;
3. sorbitan derivatives that include the TWEEN series from Uniqema, e.g.,TWEEN 60;
4. polyoxyethylene-polyoxypropylene co-polymers and block co-polymers or poloxamers, e.g., Pluronic F127, Pluronic F68 from BASF;
5. polyoxyethylene alkyl ethers, e.g., such aspolyoxyethylene glycol ethers of C₁₂-C₁₈ alcohols, e.g.,polyoxyl 10- or 20-cetyl ether or polyoxyl23-lauryl ether, or 20-oleyl ether, or polyoxyl 10-, 20- or 100-stearyl ether, as known and commerciallyavailableas the BRIJ series from Uniqema. Particularly useful products from theBRIJ series are BRIJ58; BRIJ 76; BRIJ 78; BRIJ 35, i.e., polyoxyl23 lauryl ether; and BRIJ 98, i.e., polyoxyl20 oleyl ether. These products have a m.p. betweenabout 32°C to about 43°C;
6. water-soluble tocopheryl PEG succinic acid esters available from EastmanChemical Co. with a m.p. of about 36°C, e.g, TPGS, e.g., vitamin E TPGS.
7. PEG sterol ethers having, e.g., from 5-35 [CH₂-CH,-O] units, e.g., 20-30 units,e-g., SOLULAN C24(Choleth-24 and Cetheth-24) from Chemron (Paso Robles, CA);similar products which may also be used are those which are known and commercially available as NIKKOL BPS-30 (polyethoxylated 30 phytosterol) andNIKKOL BPSH-25 (polyethoxylated 25 phytostanol) from Nikko Chemicals;
8. polyglycerol fatty acid esters, e.g., having a range of glycerol units from 4-10, or 4,6 or 10 glycerol units. For example, particularly suitable are deca-/hexa-/tetraglycerylmonostearate, e.g., DECAGLYN, HEXAGLYN and TETRAGLYN from NikkoChemicals;
9. alkylene polyol ether or ester, e.g., lauroyl macrogol-32 glycerides and/or stearoylmacrogol-32 glycerides which are GELUCIRE 44/14 and GELUCIRE 50/13 respectively;
10. polyoxyethylene mono esters of a saturated C₁₀ to C₂₂, such as C₁₈ substituted e.g. hydroxy fatty acid; e.g. 12 hydroxy stearic acid PEG ester, e.g. of PEG aboute.g. 600-900 e.g. 660 Daltons MW, e.g. SOLUTOL HS 15 from BASF (Ludwigshafen,20 Germany). According to a BASF technical leaflet MEF 151E (1986), SOLUTOL HS15 comprises about 70% polyethoxylated 12-hydroxystearate by weight and about 30% by weight unesterified polyethylene glycol component. It has a hydrogenationvalue of 90 to 110, a saponification value of 53 to 63, an acid number of maximum 1, and a maximum water content of 0.5% by weight;
11. polyoxyethylene-polyoxypropylene-alkyl ethers, e.g.polyoxyethylene-polyoxypropylene-ethers of C₁₂ to C₁₈ alcohols, e.g. polyoxyethylen-20-polyoxypropylene-4-cetylether which is commercially available as NIKKOL PBC 34 from Nikko Chemicals;
12. polyethoxylated distearates, e.g. commercially available under the tradenamesATLAS G 1821 from Uniqema and NIKKOCDS-6000P from Nikko Chemicals; and
13. lecithins, e.g. soy bean phospholipid, e.g.commercially available as LIPOID S75 from Lipoid GmbH (Ludwigshafen, Germany) or egg phospholipid, commerciallyavailable as PHOSPHOLIPON 90 from Nattermann Phospholipid (Cologne,Germany).

Examples of liquid surfactants include, but are not limited to, sorbitan derivativessuch as TWEEN 20, TWEEN 40 and TWEEN 80, SYNPERONIC L44,and polyoxyl 10-oleylether, all available from Uniqema, and polyoxyethylene containing surfactants e.g. PEG-8 caprylic/capric glycerides (e.g. Labrasolavailable from Gattefosse).

The surfactant may comprise from about 5% to about 90% by weight of the composition of the invention, e.g. from about 15% to about 85%by weight, e.g., about 20%to about 60% by weight, e.g. from about 35% to about 55% by weight.

In one aspect of the invention, the surfactant is polyoxyethylene-polyoxypropylene co-polymers and block co-polymers or poloxamers, e.g., Pluronic F127, Pluronic F68 from BASF.

In one aspect of the invention, the surfactant isa poloxamer. In a further aspect, the surfactant is selected from the group consisting of poloxamer 188, poloxamer 407and mixtures of poloxamer 407 and poloxamer 188.

In certain embodiments of the present invention, the pharmaceutical composition may comprise additional excipients commonly found in pharmaceuticalcompositions, examples of such excipients include, but are not limited to,antioxidants,antimicrobial agents, enzyme inhibitors, stabilizers, preservatives, flavors, sweeteners and other components as described in Handbook of Pharmaceutical Excipients, Rowe et al.,Eds., 4'h Edition, Pharmaceutical Press (2003), which is hereby incorporated by reference.

These additional excipients may be in an amount from about 0.05-5% by weight of the total pharmaceutical composition. Antioxidants, anti-microbial agents, enzyme inhibitors,stabilizers or preservatives typically provide up to about 0.05-1% by weight of the total pharmaceutical composition. Sweetening or flavoring agents typically provide up to about 2.5% or 5% by weight of the total pharmaceutical composition.

Examples of antioxidants include, but are not limited to, ascorbic acid and itsderivatives, tocopherol and its derivatives, butyl hydroxyl anisole and butyl hydroxyl toluene.

In one aspect of the invention, the composition comprises a buffer. The term "buffer" as used herein refers to a chemical compound in a pharmaceutical composition that reduces the tendency of pH of the composition to change over time as would otherwise occur due to chemical reactions. Buffers include chemicals such as sodium phosphate, TRIS, glycine and sodium citrate.

The term "preservative" as used herein refers to a chemical compound which is added to a pharmaceutical composition to prevent or delay microbial activity (growth and metabolism). Examples of pharmaceutically acceptable preservatives are phenol, m-cresol and a mixture of phenol and m-cresol.

The term "stabilizer" as used herein refers to chemicals added to peptide containing pharmaceutical compositions in order to stabilize the peptide, i.e. to increase the shelf life and/or in-use time of such compositions. Examples of stabilizers used in pharmaceutical formulations are L-glycine, L-histidine, arginine, glycylglycine, ethylenediamine, citrate, EDTA, zinc, sodium chloride, polyethylene glycol, carboxymethylcellulose, and surfactants and antioxidants like alfa-tocopherol and I-ascorbic acid.

Each unit dosage will suitably contain from 0.1 mg to 1000 mg polypeptide, e.g., 0.1 mg,1 mg, 5 mg, 10 mg, 15 mg, 25 mg, 50 mg, 100 mg, 200 mg, 250 mg, 300 mg, 400 mg or 500 mg, e.g., between 5 mg and 500 mg of polypeptide. In one aspect of the invention each unit dosage contains between 10 mg and 500 mg of polypeptide. In a further aspect a unit dosage form contains between 10 mg and 100 mg of polypeptide. In yet a further aspect of the invention, the unit dosage form contains between 20 mg and 500 mg of polypeptide. In yet a further aspect of the invention, the unit dosage form contains between 20 mg and 100 mg of polypeptide. Such unit dosage forms are suitable for administration 1-5 times daily depending upon the particular purpose of therapy.

In a further aspect of the present invention, a process for preparing a spontaneouslydispersible pharmaceutical composition containing a polypeptide, comprises the steps of bringing the drug and a carrier comprising a polar organic solvent, a lipophiliccomponent, a surfactant and optionally a hydrophilic component into intimate admixture. For example,the polypeptide and the carrier can be liquefied, for example, by heating to about 30°C to about 80°C, and then solidifying by cooling to room temperature.

The carrier can be prepared separately before bringing the polypeptide into intimateadmixture with the polypeptide. Alternatively, one, two or more of the components of the carrier can bemixed together with the polypeptide.

In yet a further aspect, the invention provides a process for preparing a microemulsion containing a polypeptide, which process comprises the followingsteps:
(a) bringing the polypeptide and a spontaneously dispersible preconcentrate comprising a lipophiliccomponent, a surfactant and a polar organic solvent and optionally a solid hydrophilic component into intimateadmixture to form a spontaneously dispersible pharmaceutical composition; and
(b) diluting the spontaneously dispersible pharmaceutical composition in an aqueousmedium to form a microemulsion.

In yet a further aspect, the invention provides a process for preparing a spontaneously dispersiblepreconcentrate (which can be filled into a capsule, e.g. enteric coated capsule) containing a polypeptide, which process comprises the followingsteps:
(a) dissolving the polypeptide in the polar organic solvent and
(b) mixing with the lipophilic component, surfactant and optionally hydrophilic component.

The term "therapeutically active polypeptide" or "therapeutic polypeptides" as used herein refers to a polypeptide able to cure, alleviate or partially arrest the clinical manifestations of a given disease and its complications.

In a further aspect of the invention, the term "therapeutically active polypeptide" or "therapeutic polypeptides" as used herein means a polypeptide which is being developed for therapeutic use, or which has been developed for therapeutic use.

An amount adequate to accomplish this is defined as "therapeutically effective amount".

Effective amounts for each purpose will depend on the severity of the disease or injury as well as the weight and general state of the subject. It will be understood that determining an appropriate dosage may be achieved using routine experimentation, by constructing a matrix of values and testing different points in the matrix, which is all within the ordinary skills of a trained physician or veterinary.

The therapeutically active polypeptide may be present in an amount up to about 60% such as up to about 40% by weight of the composition, or from about 0.01% such as from about 0.1%. In one aspect of the invention, the therapeutically active polypeptide may be present in an amount from about 0.01% to about 20%, in a further aspect from about 1% to 20%or from about 1% to 10% by weight of the composition. It is intended, however, that the choice of a particular level of polypeptide will be made in accordance with factors well-known in the pharmaceutical arts,including the solubility of the polypeptide in the polar organic solvent or optional hydrophilic component or surfactant used, or a mixture thereof, mode of administration and the size and condition of the patient.

The term "pharmaceutically acceptable" as used herein means suited for normal pharmaceutical applications, i.e. giving rise to no serious adverse events in patients etc.

The term "treatment of a disease" as used herein means the management and care of a patient having developed the disease, condition or disorder. The purpose of treatment is to combat the disease, condition or disorder. Treatment includes the administration of the active compounds to eliminate or control the disease, condition or disorder as well as to alleviate the symptoms or complications associated with the disease, condition or disorder, and prevention of the disease, condition or disorder.

The term "prevention of a disease" as used herein is defined as the management and care of an individual at risk of developing the disease prior to the clinical onset of the disease. The purpose of prevention is to combat the development of the disease, condition or disorder, and includes the administration of the active compounds to prevent or delay the onset of the symptoms or complications and to prevent or delay the development of related diseases, conditions or disorders.

In one aspect of the invention, the pharmaceutical formulation comprises a therapeutically active polypeptide in a concentration from 0.1 % w/w to 50 % w/w.

The term "polypeptide" or "peptide" is used interchangeably herein to mean a compound composed of at least five constituent amino acids connected by peptide bonds. The constituent amino acids may be from the group of the amino acids encoded by the genetic code and they may be natural amino acids which are not encoded by the genetic code, as well as synthetic amino acids. Natural amino acids which are not encoded by the genetic code are e.g. hydroxyproline, γ-carboxyglutamate, ornithine, phosphoserine, D-alanine and D-glutamine. Synthetic amino acids comprise amino acids manufactured by chemical synthesis, i.e. D-isomers of the amino acids encoded by the genetic code such as D-alanine and D-leucine, Aib (α-aminoisobutyric acid), Abu (α-aminobutyric acid), Tle (tert-butylglycine), β-alanine, 3-aminomethyl benzoic acid, anthranilic acid.

The production of polypeptides and peptides is well known in the art. Polypeptides or peptides may for instance be produced by classical peptide synthesis, e.g. solid phase peptide synthesis using t-Boc or Fmoc chemistry or other well established techniques, see e.g. Greene and Wuts, "Protective Groups in Organic Synthesis", John Wiley & Sons, 1999. The polypeptides or peptides may also be produced by a method which comprises culturing a host cell containing a DNA sequence encoding the (poly)peptide and capable of expressing the (poly)peptide in a suitable nutrient medium under conditions permitting the expression of the peptide. For (poly)peptides comprising non-natural amino acid residues, the recombinant cell should be modified such that the non-natural amino acids are incorporated into the (poly)peptide, for instance by use of tRNA mutants.

In one aspect of the invention, a therapeutically active polypeptide according to the invention is water soluble. As used herein, the term water soluble polypeptide refers to polypeptides which can be dissolved at RT in a concentration of at least 10% in demineralised water, at a pH of at least 2 pH units away from its isoelectric point.

In one aspect of the invention, the water solubility is at least 100 mg/ml. In a further aspect, the water solubility is at least 120 mg/ml. In yet a further aspect, the water solubility is at least 140 mg/ml. In yet a further aspect, the water solubility is at least 160 mg/ml. In yet a further aspect, the water solubility is at least 180 mg/ml. In yet a further aspect, the water solubility is at least 200 mg/ml. In yet a further aspect, the water solubility is at least 250 mg/ml. In yet a further aspect, the water solubility is at least 300 mg/ml.

The term "isoelectric point" as used herein means the pH value where the overall net charge of a macromolecule such as a peptide is zero. In peptides there may be several charged groups, and at the isoelectric point the sum of all these charges is zero. At a pH above the isoelectric point the overall net charge of the peptide will be negative, whereas at pH values below the isoelectric point the overall net charge of the peptide will be positive.

The term "analogue" as used herein referring to a peptide means a modified peptide wherein one or more amino acid residues of the peptide have been substituted by other amino acid residues and/or wherein one or more amino acid residues have been deleted from the peptide and/or wherein one or more amino acid residues have been added to the peptide. Such addition or deletion of amino acid residues can take place at the N-terminal of the peptide and/or at the C-terminal of the peptide. In one embodiment an analogue comprises less than 8 modifications (substitutions, deletions, additions) relative to the native peptide. In one embodiment an analogue comprises less than 7 modifications (substitutions, deletions, additions) relative to the native peptide. In one embodiment an analogue comprises less than 6 modifications (substitutions, deletions, additions) relative to the native peptide. In another embodiment an analogue comprises less than 5 modifications (substitutions, deletions, additions) relative to the native peptide. In another embodiment an analogue comprises less than 4 modifications (substitutions, deletions, additions) relative to the native peptide. In another embodiment an analogue comprises less than 3 modifications (substitutions, deletions, additions) relative to the native peptide. In another embodiment an analogue comprises less than 2 modifications (substitutions, deletions, additions) relative to the native peptide. In another embodiment an analogue comprises only a single modification (substitutions, deletions, additions) relative to the native peptide. The added and/or exchanged amino acid residues can either be codable amino acid residues or other naturally occurring residues or purely synthetic amino acid residues.

The term "derivative" as used herein in relation to a parent peptide means a chemically modified parent protein or an analogue thereof, wherein at least one substituent is not present in the parent protein or an analogue thereof, i.e. a parent protein which has been covalently modified. Typical modifications are amides, carbohydrates, alkyl groups, acyl groups, esters, PEGylations and the like.

The term "GLP-1 compound" as used herein means GLP-1(7-37) (SEQ ID NO. 1), insulinotropic analogue thereof and insulinotropic derivatives thereof. Non-limiting examples of GLP-1 analogues are GLP-1(7-36) amide, Arg³⁴-GLP-1(7-37), Gly⁸-GLP-1(7-37), Val⁸-GLP-1(7-36)-amide and Val⁸Asp²²-GLP-1(7-37). Non-limiting examples of GLP-1 derivatives are desamino-His⁷, Arg²⁶, Lys³⁴(N^{ε}-(γ-Glu(N^{α}-hexadecanoyl)))-GLP-1(7-37), desamino-His⁷, Arg²⁶, Lys³⁴(N^{ε}-octanoyl)-GLP-1(7-37), Arg^{26,34}, Lys³⁸(N^{ε}-(ω-carboxypentadecanoyl))-GLP-1(7-38), Arg^{26,34}, Lys³⁶(N^{ε}-(γ-Glu(N^{α}-hexadecanoyl)))-GLP-1(7-36) and Arg³⁴, Lys²⁶(N^{ε}-(γ-Glu(N^{α}-hexadecanoyl)))-GLP-1(7-37);N-epsilon26--(17-carboxyheptadecanoyl)-[Aib8,Arg34]GLP-1-(7-37)-peptide; N-epsilon26-(19-carboxynonadecanoyl)-[Aib8,Arg34]GLP-1-(7-37)-peptide; N-epsilon26-(4-{[N-(2-carboxyethyl)-N-(15-carboxypentadecanoyl)amino]methyl}benzoyl)[Arg34]GLP-1-(7-37); N-epsilon26-[2-(2-[2-(2-[2-(2-[4-(17-Carboxy-heptadecanoylamino)-4(S)-carboxybutyrylamino]ethoxy)ethoxy]acetylamino)ethoxy]ethoxy)acetyl][Aib8,Ar g34]GLP-1-(7-37)peptide; N-epsilon37{2-[2-(2-{2-[2-((R)-3-carboxy-3-{[1-(19-carboxynonadecanoyl)piperidine-4-carbonyl]amino}propionylamino)ethoxy]ethoxy}acetylamino)ethoxy]ethoxy}ace tyl [desaminoHis7,Glu22,Arg26,Arg34,Lys37]GLP-1(7-37)amide; N-epsilon37{2-[2-(2-{2-[2-((S)-3-carboxy-3-{[1-(19-carboxynonadecanoyl)piperidine-4-carbonyl]amino}propionylamino)ethoxy]ethoxy}acetylamino)ethoxy]ethoxy} acetyl Aib8,Glu22,Arg26,Arg34,Lys37]GLP-1-(7-37)amide; N-epsilon37-[2-(2-[2-(2-[2-(2-((R)-3-[1-(17-Carboxyheptadecanoyl)piperidin-4-ylcarbonyl-amino]3-carboxypropionylamino)ethoxy)ethoxy]acetylamino) ethoxy]ethoxy)acetyl][DesaminoHis7, Glu22 Arg26, Arg 34, Phe(m-CF3)28]GLP-1-(7-37)amide; N-epsilon30{2-[2-(2-{2-[2-((S)-3-carboxy-3-{[1-(19-carboxynonadecanoyl)piperidine-4-carbonyl]amino}propionylamino)ethoxy]ethoxy}acetylamino)ethoxy]ethoxy} acetyl [Aib8,Glu22,Arg26,Lys30]GLP-1-(7-37); N-epsilon31{2-[2-(2-{2-[2-((S)-3-carboxy-3-{[1-(19-carboxynona-decanoyl)piperidine-4-carbonyl]amino}propionylamino)ethoxy]ethoxy}acetylamino)ethoxy]ethoxy} acetyl [Aib8, Glu22, Arg26,Lys 31]GLP-1-(7-37); N-epsilon31-(2-{2-[2-(2-{2-[2-((S)-3-Carboxy-3-{[1-(19-carboxy-nonadecanoyl)piperidine-4-carbonyl]amino}propionylamino)ethoxy]ethoxy}acetylamino)ethoxy]ethoxy}ace tyl) [Aib8,Glu22,Arg26,Lys31,Arg34]GLP-1-(7-37);N-epsilon37-[2-(2-{2-[2-(2-{2-[(S)-4-Carboxy-4-({trans-4-[(19-carboxy-nonadecanoyl-amino)methyl]cyclohexanecarbonyl}amino)butyrylamino]ethoxy}ethoxy)acetyla mino] ethoxy}ethoxy)acetyl][Aib8,Glu22,Arg26,Arg34,Lys37]GLP-1-(7-37)amide; N-epsilon37-[2-(2-{2-[2-(2-{2-[(S)-4-Carboxy-4-({trans-4-[(19-carboxy-nonadecanoylamino)methyl]cyclohexanecarbonyl}amino)butyrylamino]ethoxy}e thoxy) acetylamino]ethoxy}ethoxy)acetyl][DesaminoHis7,Glu22,Arg26,Arg34,Lys37]GL P-1-(7-37)amide; N-epsilon37-[2-(2-{2-[2-(2-{2-[(S)-4-Carboxy-4-({trans-4-[(19-carboxy-nonadecanoyl-amino)methyl]cyclohexanecarbonyl}amino)butyrylamino]ethoxy}ethoxy)acetyla mino] ethoxy}ethoxy)acetyl][DesaminoHis7,Glu22,Arg26, Arg34,Lys37]GLP-1-(7-37); N-epsilon37-[2-(2-{2-[2-(2-{2-[(S)-4-Carboxy-4-({trans-4-[(19-carboxy-nonadecanoylamino)methyl]cyclohexanecarbonyl}amino)butyrylamino] ethoxy}ethoxy)acetylamino]ethoxy}ethoxy)acetyl][DesaminoHis7,Glu22,Arg26, Glu30,Arg34, Lys37]GLP-1-(7-37); N-epsilon20-[2-(2-{2-[(S)-4-Carboxy-4-((S)-4-carboxy-4-{12-[4-(16-(1H-tetrazol-5-yl)hexadecanoyl-sulfamoyl)butyrylamino]dodecanoylamino}butyrylamino)butyrylamino]ethoxy}et hoxy)acetyl][Aib8,Lys20,Glu22,Arg26,Glu30,Pro37]GLP-1-(7-37)amide; N-epsilon37-[2-(2-{2-[(S)-4-Carboxy-4-((S)-4-carboxy-4-{12-[4-(16-(1H-tetrazol-5-yl)hexadecanoylsulfamoyl)butyrylamino]dodecanoylamino}butyrylamino)butyryl amino]ethoxy}ethoxy)acetyl][Aib8,Glu22,Arg26,Arg34,Lys37]GLP-1-(7-37)amide; N-epsilon37-[2-(2-{2-[(S)-4-Carboxy-4-((S)-4-carboxy-4-{12-[4-(16-(1H-tetrazol-5-yl)hexadecanoylsulfamoyl)butyrylamino]dodecanoylamino}butyrylamino)butyryl amino]ethoxy}ethoxy)acetyl][DesaminoHis7,Glu22,Arg26,Arg34,Lys37]GLP-1-(7-37)amide; [Aib8,Glu22,Arg26,Glu30,Pro37]GLP-1-(7-37)Lys [2-(2-{2-[4-Carboxy-4-(4-carboxy-4-{4-[4-(16-1H-tetrazol-5-yl-hexadecanoylsulfamoyl)butyrylamino]butyrylamino} butyrylamino)butyrylamino]ethoxy}ethoxy)acetyl]; N-epsilon37 (Polyethyleneglycol2000)[DesaminoHis7,Glu22,Arg26,Arg34,Lys37]GLP-1 (7-37) amide; N-epsilon37 (3-((2-(2-(2-(2-(2-Hexadecyloxy-ethoxy)ethoxy)ethoxy)ethoxy)ethoxy)) propionyl) [DesaminoHis7,Glu22,Arg26,Arg34,Lys37]GLP-1(7-37)-amide; N-epsilon37-{2-(2-(2-(2-[2-(2-(4-(hexadecanoylamino)-4-carboxybutyrylamino)ethoxy) ethoxy]acetyl)ethoxy)ethoxy)acetyl)}-[desaminoHis7,Glu22,Arg26, Glu30,Arg34,Lys37] (GLP-1-(7-37)amide; N-epsilon37-{2-(2-(2-(2-[2-(2-(4-(hexadecanoylamino)-4-carboxybutyrylamino)ethoxy) ethoxy]acetyl)ethoxy)ethoxy)acetyl)}-[desaminoHis7,Glu22, Arg26,Arg34,Lys 37] (GLP-1-(7-37)amide; N-epsilon37-(2-(2-(2-(2-(2-(2-(2-(2-(2-Octadecanoylamino)ethoxy)ethoxy)acetylamino) ethoxy)ethoxy) acetylamino)ethoxy)ethoxy)acetyl) [desaminoHis7,Glu22,Arg26,Arg34,Lys37] GLP-1 (7-37)amide; N-epsilon36-(2-(2-(2-((2-[2-(2-(17-carboxyheptadecanoylamino)ethoxy)ethoxy] acetylamino)ethoxy)ethoxy)acetyl) [Aib8,Glu22,Arg26,Glu30,Lys36] GLP-1-(7-37)Glu-amide; N-epsilon37-[4-(16-(1H-Tetrazol-5-yl)hexadecanoylsulfamoyl)butyryl] [DesaminoHis7,Glu22,Arg26,Arg34,Lys37]GLP-1-(7-37)amide; N-epsilon37-[2-(2-{2-[2-(2-{2-[(S)-4-Carboxy-4-(19-carboxy-nonadecanoyl amino)butyrylamino]ethoxy}ethoxy)acetylamino]ethoxy}ethoxy)acetyl][Desami noHis7,Glu22,Arg26,Arg34,Lys37]GLP-1-(7-37); and N-epsilon31-[2-(2-{2-[2-(2-{2-[4-Carboxy-4-(17-carboxy-heptadecanoylamino) butyrylamino]ethoxy}ethoxy)acetylamino]ethoxy}ethoxy)acetyl][Aib8,Glu22,Arg 26,Lys31]GLP-1-(7-37).

The term "dipeptidyl aminopeptidase IV protected" as used herein means a compound, e.g. a GLP-1 analogue, which is more resistant to dipeptidyl aminopeptidase IV (DPP-IV) than the native compound, e.g. GLP-1(7-37). Resistance of a GLP-1 compound towards degradation by dipeptidyl aminopeptidase IV is determined by the following degradation assay:

Aliquots of the GLP-1 compound (5 nmol) are incubated at 37 °C with 1 µL of purified dipeptidyl aminopeptidase IV corresponding to an enzymatic activity of 5 mU for 10-180 minutes in 100 µL of 0.1 M triethylamine-HCl buffer, pH 7.4. Enzymatic reactions are terminated by the addition of 5 µL of 10% trifluoroacetic acid, and the peptide degradation products are separated and quantified using HPLC analysis. One method for performing this analysis is : The mixtures are applied onto a Vydac C18 widepore (30 nm pores, 5 pm particles) 250 x 4.6 mm column and eluted at a flow rate of 1 ml/min with linear stepwise gradients of acetonitrile in 0.1% trifluoroacetic acid (0% acetonitrile for 3 min, 0-24% acetonitrile for 17 min, 24-48% acetonitrile for 1 min) according to Siegel et al., Regul. Pept. 1999;79:93-102 and Mentlein et al. Eur. J. Biochem. 1993;214:829-35. Peptides and their degradation products may be monitored by their absorbance at 220 nm (peptide bonds) or 280 nm (aromatic amino acids), and are quantified by integration of their peak areas related to those of standards. The rate of hydrolysis of a GLP-1 compound by dipeptidyl aminopeptidase IV is estimated at incubation times which result in less than 10% of the GLP-1 compound being hydrolysed.

The term "insulinotropic" as used herein referring to a peptide or a compound means the ability to stimulate secretion of insulin in response to an increased plasma glucose level. Insulinotropic peptides and compounds are agonists of the GLP-1 receptor. The insulinotropic property of a compound may be determined by in vitro or in vivo assays known in the art. The following in vitro assay may be used to determine the insulinotropic nature of a compound such as a peptide. Preferably insulinotropic compounds exhibit an EC₅₀ value in below assay of less than 5 nM, even more preferably EC50 values less than 500 pM.

Baby hamster kidney (BHK) cells expressing the cloned human GLP-1 receptor (BHK 467-12A) are grown in DMEM media with the addition of 100 IU/mL penicillin, 100 µL/mL streptomycin, 10% foetal calf serum and 1 mg/mL Geneticin G-418 (Life Technologies). Plasma membranes are prepared by homogenization in buffer (10 mM Tris-HCl, 30 mM NaCl and 1 mM dithiothreitol, pH 7.4, containing, in addition, 5 mg/mL leupeptin (Sigma), 5 mg/L pepstatin (Sigma), 100 mg/L bacitracin (Sigma), and 16 mg/L aprotinin (Calbiochem-Novabiochem, La Jolla, CA)). The homogenate was centrifuged on top of a layer of 41% W7v sucrose. The white band between the two layers was diluted in buffer and centrifuged. Plasma membranes were stored at -80 °C until used.

The functional receptor assay is carried out by measuring cAMP as a response to stimulation by the insulinotropic peptide or insulinotropic compound. Incubations are carried out in 96-well microtiter plates in a total volume of 140 mL and with the following final concentrations: 50 mM Tris-HCl, 1 mM EGTA, 1.5 mM MgSO₄, 1.7 mM ATP, 20 mM GTP, 2 mM 3-isobutyl-1-methylxanthine (IBMX), 0.01% w/v Tween-20, pH 7.4. Compounds are dissolved and diluted in buffer. GTP is freshly prepared for each experiment: 2.5 µg of membrane is added to each well and the mixture is incubated for 90 min at room temperature in the dark with shaking. The reaction is stopped by the addition of 25 mL 0.5 M HCl. Formed cAMP is measured by a scintillation proximity assay (RPA 542, Amersham, UK). A dose-response curve is plotted for the compound and the EC₅₀ value is calculated using GraphPad Prism software.

The term "prodrug of an insulinotropic compound" as used herein means a chemically modified compound which following administration to the patient is converted to an insulinotropic compound. Such prodrugs are typically amino acid extended versions or esters of an insulinotropic compound.

The term "exendin-4 compound" as used herein is defined as exendin-4(1-39) (SEQ ID NO. 2), insulinotropic fragments thereof, insulinotropic analogs thereof and insulinotropic derivatives thereof. Insulinotropic fragments of exendin-4 are insulinotropic peptides for which the entire sequence can be found in the sequence of exendin-4 (SEQ ID NO. 2) and where at least one terminal amino acid has been deleted. Examples of insulinotropic fragments of exendin-4(1-39) are exendin-4(1-38) and exendin-4(1-31). The insulinotropic property of a compound may be determined by in vivo or in vitro assays well known in the art. For instance, the compound may be administered to an animal and monitoring the insulin concentration over time. Insulinotropic analogs of exendin-4(1-39) refer to the respective molecules wherein one or more of the amino acids residues have been exchanged with other amino acid residues and/or from which one or more amino acid residues have been deleted and/or from which one or more amino acid residues have been added with the proviso that said analogue either is insulinotropic or is a prodrug of an insulinotropic compound. An example of an insulinotropic analog of exendin-4(1-39) is Ser²Asp³-exendin-4(1-39) wherein the amino acid residues in position 2 and 3 have been replaced with serine and aspartic acid, respectively (this particular analog also being known in the art as exendin-3). Insulinotropic derivatives of exendin-4(1-39) and analogs thereof are what the person skilled in the art considers to be derivatives of these peptides, i.e. having at least one substituent which is not present in the parent peptide molecule with the proviso that said derivative either is insulinotropic or is a prodrug of an insulinotropic compound. Examples of substituents are amides, carbohydrates, alkyl groups, esters and lipophilic substituents. An example of an insulinotropic derivative of exendin-4(1-39) and analogs thereof is Tyr³¹-exendin-4(1-31)-amide.

The term "stable exendin-4 compound" as used herein means a chemically modified exendin-4(1-39), i.e. an analogue or a derivative which exhibits an in vivo plasma elimination half-life of at least 10 hours in man, as determined by conventional methods.

The term "dipeptidyl aminopeptidase IV protected exendin-4 compound" as used herein means an exendin-4 compound which is more resistant towards the plasma peptidase dipeptidyl aminopeptidase IV (DPP-IV) than exendin-4 (SEQ ID NO. 2), as determined by the assay described under the definition of dipeptidyl aminopeptidase IV protected GLP-1 compound.

Human insulin consists of two polypeptide chains, the A and B chains which contain 21 and 30 amino acid residues, respectively. The A and B chains are interconnected by two disulphide bridges. Insulin from most other species is similar, but may contain amino acid substitutions in some positions.

With "insulin peptide" as used herein is meant human insulin, porcine insulin or bovine insulin with disulfide bridges between CysA7 and CysB7 and between CysA20 and CysB19 and an internal disulfide bridge between CysA6 and CysA11 or an insulin analogue or derivative thereof.

An insulin analogue as used herein is a polypeptide which has a molecular structure which formally can be derived from the structure of a naturally occurring insulin, for example that of human insulin, by deleting and/or substituting at least one amino acid residue occurring in the natural insulin and/or by adding at least one amino acid residue.

In one aspect an insulin analogue according to the invention comprises less than 8 modifications (substitutions, deletions, additions) relative to human insulin. In one aspect an insulin analogue comprises less than 7 modifications (substitutions, deletions, additions) relative to human insulin. In one aspect an insulin analogue comprises less than 6 modifications (substitutions, deletions, additions) relative to human insulin. In another aspect an insulin analogue comprises less than 5 modifications (substitutions, deletions, additions) relative to human insulin. In another aspect an insulin analogue comprises less than 4 modifications (substitutions, deletions, additions) relative to human insulin. In another aspect an insulin analogue comprises less than 3 modifications (substitutions, deletions, additions) relative to human insulin. In another aspect an insulin analogue comprises less than 2 modifications (substitutions, deletions, additions) relative to human insulin.

The insulin analogues may be such that e.g. a fast onset of action, a protracted action and/or a stability towards proteases is obtained.

In one aspect the insulin analogues are such that a fast onset of action is obtained, i.e. the onset of action of the insulin analogue is within 4 hours, alternatively 3 hours, 2 hours, 1 hour or ½ hour after administration. In another aspect the insulin analogues are such that a protracted action is obtained, i.e. the action of the insulin analogue is continued for more than 4 hours, alternatively 6 hours, 8 hours, 12 hours, 18 hours or 24 hours after administration.

The insulin analogues may be such wherein position 28 of the B chain may be modified from the natural Pro residue to one of Asp, Lys, Leu, Val, Ala or Ile. In another aspect Lys at position B29 of insulin is modified to Pro or Glu. In one aspect an insulin analogue according to the invention is such wherein the amino acid residue in position B28 of insulin is Pro, Asp, Lys, Leu, Val, or Ala and the amino acid residue in position B29 is Lys or Pro and optionally the amino acid residue in position B30 is deleted. Also, Asn at position A21 may be modified to Ala, Gln, Glu, Gly, His, Ile, Leu, Met, Ser, Thr, Trp, Tyr or Val, in particular to Gly, Ala, Ser, or Thr and preferably to Gly. Furthermore, Asn at position B3 may be modified to Lys,Thr, Ser, Gln, Glu or Asp. Further examples of insulin analogues are des(B30) human insulin; des(B30) human insulin analogues; insulin analogues wherein PheB1 has been deleted; insulin analogues wherein the A-chain and/or the B-chain have an N-terminal extension and insulin analogues wherein the A-chain and/or the B-chain have a C-terminal extension. Thus one or two Arg may be added to position B1. In another aspect an insulin analogue according to the invention is des(B28-B30) human insulin, des(B27) human insulin or des(B30) human insulin. In yet another aspect an insulin analogue according to the invention is an insulin analogue wherein the amino acid residue in position B3 is Lys and the amino acid residue in position B29 is Glu or Asp.

In another aspect an insulin analogue according to the invention is des(B28-B30) human insulin, des(B27) human insulin or des(B30) human insulin. In yet another aspect an insulin analogue according to the invention is an insulin analogue wherein the amino acid residue in position B3 is Lys and the amino acid residue in position B29 is Glu or Asp.

In another aspect the insulin peptide is an insulin analogue selected from the group consisting of AspB28 human insulin; LysB28ProB29 human insulin; LysB3GluB29 human insulin and A14GluB25HisdesB30 human insulin.

Insulin analogues according to the invention may be protected towards degradation by proteases as e.g. described in WO 2008/034881 (Novo Nordisk).

In one aspect an insulin analogue according to the invention is an insulin analogue which is protease protected.

In one aspect of the invention a protease protected insulin analogue is an insulin analogue wherein the amino acid in position A14 is Glu or His, the amino acid in position B25 is His and which optionally further comprises one or more additional mutations;
an insulin analogue wherein
● the amino acid in position A8 is His and/or the amino acid in position A12 is Glu or Asp and/or the amino acid in position A13 is His, Asn, Glu or Asp and/or the amino acid in position A14 is Asn, Gln, Glu, Arg, Asp, Gly or His and/or the amino acid in position A15 is Glu or Asp; and
● the amino acid in position B1 is Glu and/or the amino acid in position B16 is Glu or His and or the amino acid in position B25 is His and/or the amino acid in position B26 is His, Gly, Asp or Thr and/or the amino acid in position B27 is His, Glu, Lys, Gly or Arg and/or the amino acid in position B28 is His, Gly or Asp; and
which optionally further comprises one or more additional mutations; or
an insulin analogue wherein the amino acid in position A14 isselected from the group consisting of Lys, Glu, Arg, Asp, Pro and His; and the B-chain of the insulin analogue comprises at least two mutations relative to the parent insulin, wherein two or more mutations are in the form of deletions of the amino acids in positions B27, B28, B29 and B30, or a combination of a deletion of the amino acid in position B30 and a substitution of an amino acid selected from the amino acid substitutions in position: B25 to His, B26 to Gly or Glu, B27 to Gly or Lys and B28 to Asp, His, Gly, Lys or Glu.

In a yet further aspect an insulin of the invention is selected from the group consisting of: human insulin; DesB30 human insulin; AspB28 human insulin; AspB28,DesB30 human insulin; LysB3,GluB29 human insulin; LysB28,ProB29 human insulin; GluA14,HisB25 human insulin; HisA14,HisB25 human insulin; GluA14,HisB25,DesB30 human insulin; HisA14, HisB25,DesB30 human insulin; GluA14,HisB25,desB27,desB28,desB29,desB30 human insulin; GluA14,HisB25,GluB27,desB30 human insulin; GluA14,HisB16,HisB25,desB30 human insulin; HisA14,HisB16,HisB25,desB30 human insulin; HisA8,GluA14,HisB25,GluB27,desB30 human insulin; HisA8,GluA14,GluB1,GluB16,HisB25,GluB27,desB30 human insulin; and HisA8,GluA14,GluB16,HisB25,desB30 human insulin.

The GLP-1 analogues may be such wherein the naturally occurring Lys at position 34 of GLP-1(7-37) has been substituted with Arg.

All amino acids for which the optical isomer is not stated is to be understood to mean the L-isomer.

In aspects of the invention a maximum of 17 amino acids have been modified. In aspects of the invention a maximum of 15 amino acids have been modified. In aspects of the invention a maximum of 10 amino acids have been modified. In aspects of the invention a maximum of 8 amino acids have been modified. In aspects of the invention a maximum of 7 amino acids have been modified. In aspects of the invention a maximum of 6 amino acids have been modified. In aspects of the invention a maximum of 5 amino acids have been modified. In aspects of the invention a maximum of 4 amino acids have been modified. In aspects of the invention a maximum of 3 amino acids have been modified. In aspects of the invention a maximum of 2 amino acids have been modified. In aspects of the invention 1 amino acid has been modified.

With "desB30 insulin", "desB30 human insulin"is meant insulin or an analogue thereof lacking the B30 amino acid residue.

By "parent insulin" is meant a naturally occurring insulin such as human insulin or porcine insulin. Alternatively, the parent insulin can be an insulin analogue.

In one aspect of the present invention, the therapeutically active polypeptide is an insulin peptide.

In one aspect of the invention, the insulin peptide is human insulin, an analog of human insulin, a derivative of human insulin or a derivative of a human insulin analog.

In one aspect of the invention, the insulin peptide is human insulin.

In one aspect of the invention, the insulin peptide is an insulin derivative. In a further aspect of the invention, the insulin derivative is selected from the group consisting of B29-N^{ε}-myristoyl-des(B30) human insulin, B29-N^{ε}-palmitoyl-des(B30) human insulin, B29-N^{ε}-myristoyl human insulin, B29-N^{ε}-palmitoyl human insulin, B28-N^{ε}-myristoyl Lys^{B28} Pro^{B29} human insulin, B28-N^{ε}-palmitoyl Lys^{B28} Pro^{B29} human insulin, B30-N^{ε}-myristoyl-Thr^{B29}Lys^{B30} human insulin, B30-N^{ε}-palmitoyl-Thr^{B29}Lys^{B30} human insulin, B29-N^{ε}-(N-palmitoyl-γ-glutamyl)-des(B30) human insulin, B29-N^{ε}-(N-lithocholyl-γ-glutamyl)-des(B30) human insulin, B29-N^{ε}-(ω-carboxyheptadecanoyl)-des(B30) human insulin and B29-N^{ε}-(ω-carboxyheptadecanoyl) human insulin.

In another aspect of the invention, the insulin derivative is B29-N^{ε}-myristoyl-des(B30) human insulin.

In a further aspect of the invention the insulin peptide is acid-stabilised insulin.

The acid-stabilised insulin may be selected from analogues of human insulin having one of the following amino acid residue substitutions:
A21G
A21G, B28K, B29P
A21G, B28D
A21G, B28E
A21G, B3K, B29E
A21G, desB27
A21G, B9E
A21G, B9D
A21G, B10E.

In a further aspect of the invention, the insulin peptide is an insulin analogue. The insulin analogue may be selected from the group consisting of an analogue wherein position B28 is Asp, Lys, Leu, Val, or Ala and position B29 is Lys or Pro; and des(B28-B30), des(B27) or des(B30) human insulin.

In another aspect of the invention, the insulin analogue is an analogue of human insulin wherein position B28 is Asp or Lys, and position B29 is Lys or Pro.

In another aspect of the invention, the insulin analogue is des(B30) human insulin.

In another aspect of the invention, the insulin analogue is an analogue of human insulin wherein position B28 is Asp.

In another aspect of the invention, the insulin analogue is an analogue wherein position B3 is Lys and position B29 is Glu or Asp.

In another aspect of the invention, the insulin analogs and derivatives are selected from among those disclosed in EP 0 792 290 (Novo Nordisk A/S), EP 0 214 826 and EP 0 705 275 (Novo Nordisk A/S), US 5,504,188 (Eli Lilly), EP 0 368 187 (Aventis), US patents 5,750,497 and 6,011,007, EP 375437 and EP 383472 and where such insulins may include, but are not limited to, insulin glulisine ( also known as Apidra®, differs from human insulin in that the amino acid asparagine at position B3 is replaced by lysine and the lysine in position B29 is replaced by glutamic acid), Lys^{B28} Pro^{B29} human insulin (Humalog®) , and Asp^{B28} human insulin (insulin aspart (Novolog®)).

In one aspect of the invention, said human insulin analog is Asp^{B28}-human insulin. In another aspect of the invention, said human insulin analog is Lys^{B28},Pro^{B29}-human insulin. In another aspect of the invention, said human insulin analog is Lys^{B3},Glu^{B29}-human insulin (insulin glulisine). In another aspect of the invention, said human insulin analog is des(B30) human insulin.

Also, derivatives of precursors or intermediates are covered by the invention. An example of such a derivative is a single-chain insulin which comprises the B- and the A-chain of human insulin or analogues or derivatives thereof connected by a connecting peptide.

An insulin derivative according to the invention is a naturally occurring insulin or an insulin analogue which has been chemically modified, e.g. by introducing a side chain in one or more positions of the insulin backbone or by oxidizing or reducing groups of the amino acid residues in the insulin or by converting a free carboxylic group to an ester group or to an amide group. Other derivatives are obtained by acylating a free amino group or a hydroxy group, such as in the B29 position of human insulin or desB30 human insulin. A non-limiting example of acylated polypeptides may e.g. be found in WO 95/07931 which is are hereby incorporated by reference.

In an aspect of the invention, the therapeutically active polypeptide is selected from the group consisting of single chain insulin (such as e.g. described in WO 2005/054291), insulinotropic peptide, GLP-1(7-37) or an analog or derivative thereof, insulin mimetics (such as e.g. described in WO 2006/018450), exendin or an analog or derivative thereof, GLP-2 or an analog or derivative thereof, a polypeptide that binds to the MC4 receptor, human growth hormone or an analog thereof, factor VII or an analog thereof, parathyroid hormone or an analog thereof, human follicle stimulating hormone or an analog thereof, a growth factor such as platelet-derived growth factor (PDGF), Obestatin, transforming growth factor α (TGF-α), transforming growth factor β (TGF-β), epidermal growth factor (EGF), vascular endothelial growth factor (VEGF), a somatomedin such as insulin growth factor I (IGF-I), insulin growth factor II (IFG-II), erythropoietin (EPO), thrombopoietin (TPO) or angiopoietin, interferon, pro-urokinase, urokinase, tissue plasminogen activator (t-PA), plasminogen activator inhibitor 1, plasminogen activator inhibitor 2, von Willebrandt factor, a cytokine, e.g. an interleukin such as interleukin (IL) 1, IL-1Ra, IL-2, IL-4, IL-5, IL-6, IL-9, IL-11, IL-12, IL-13, IL-15, IL-16, IL-17, IL-18, IL-20 or IL-21, a colony stimulating factor (CFS) such as GM-CSF, stem cell factor, a tumor necrosis factor such as TNF-α, lymphotoxin-α, lymphotoxin-β, CD40L, or CD30L, a protease inhibitor e.g. aprotinin, an enzyme such as superoxide dismutase, asparaginase, arginase, arginine deaminase, adenosine deaminase, ribonuclease, catalase, uricase, bilirubin oxidase, trypsin, papain, alkaline phosphatase, β-glucoronidase, purine nucleoside phosphorylase or batroxobin, an opioid, e.g. endorphins, enkephalins or non-natural opioids, a hormone or neuropeptide, e.g. calcitonin, glucagon, gastrins, adrenocorticotropic hormone (ACTH), cholecystokinins, lutenizing hormone, gonadotropin-releassing hormone, chorionic gonadotropin, corticotrophin-releasing factor, vasopressin, oxytocin, antidiuretic hormones, thyroid-stimulating hormone, thyrotropin-releasing hormone, relaxin, prolactin, peptide YY, neuropeptide Y, pancreastic polypeptide, leptin, CART (cocaine and amphetamine regulated transcript), a CART related peptide, perilipin, peptide hormones acting on the melanocortin receptors such as α-MSH or ACTH, melanin-concentrating hormones, natriuretic peptides, adrenomedullin, endothelin, secretin, amylin, vasoactive intestinal peptide (VIP), pituary adenylate cyclase activating polypeptide (PACAP), bombesin, bombesin-like peptides, thymosin, heparin-binding protein, soluble CD4, hypothalmic releasing factor, melanotonins and analogs thereof.

Conformational stability protein based drugs is important for maintaining biological activity and for minimizing irreversible loss of structure due to denaturation and fibrillation. Especially large polypeptides and proteins are labile with respect to conformational change due to complicated refolding patterns. Also, polypeptides with a known history of fibrillation, such as glucagon, GLP-1, insulin and amylin, are particularly sensitive towards destabilization of tertiary structure (i.e. formation of a molten globular state).

In one aspect of the invention, the therapeutically active polypeptide has a molar weight of less than 100 kDa, less than 50 kDa, or less than 10 kDa.

In another aspect of the invention, the therapeutically active polypeptide comprises less than 100 amino acids, or less than 90 amino acids, or less than 60 amino acids. In another aspect of the invention, the therapeutically active polypeptide comprises at least 10 amino acids, at least 15 amino acids, or at least 20 amino acids. In a further aspect of the invention, the therapeutically active polypeptide comprises 10 - 100 amino acids, in a further aspect 15 - 90 amino acids, in a further aspect 20-80 amino acids, in a further aspect 20 - 70 amino acids, in a further aspect 25 - 70 amino acids, in yet a further aspect 25 - 65 amino acids, in yet a further aspect 25 - 60 amino acids or 25 - 55 amino acids. In a yet further aspect, the therapeutically active polypeptide comprises 30 - 70 amino acids, 30 - 65 amino acids, 30 - 60 amino acids or 30 - 55 amino acids.

In order to increase the shelf-stability of the pharmaceutical composition it has been found that solidifying is advantageously. It is believed that the increased shelf stability is due to fewertendencies of the polypeptides to fibrillate in a solidified pharmaceutical composition.

The term "shelf-stable pharmaceutical composition" as used hereinmeans a pharmaceutical composition which is stable for at least the period which is required by regulatory agencies in connection with therapeutic proteins. Preferably, a shelf-stable pharmaceutical composition is stable for at least one year at 5 °C. Shelf-stability includes chemical stability as well as physicalstability. Chemical instability involves degradation of covalent bonds, such as hydrolysis, racemization, oxidation or crosslinking.Chemical stability of the formulations is evaluated by means ofreverse phase (RP-HPLC) and size exclusion chromatographySE-HPLC). In one aspect of the invention, the formation of peptiderelated impurities during shelf-life is less than 20 % of the totalpeptide content. In a further aspect of the invention, the formation of peptide related during impurities during shelf-life isless than 10 %. In a further aspect of the invention, the formation of peptide related during impurities during shelf-life isless than 5 %. The RP-HPLC analysis is typically conducted inwater-acetonitrile or water-ethanol mixtures. In one embodiment,the solvent in the RP-HPLC step will comprise a salt such as Na₂SO₄, (NH₄)₂SO₄, NaCI, KCI, and buffer systems such as phosphate,and citrate and maleic acid. The required concentration of salt in the solvent may be from about 0.1 M to about 1 M, preferablebetween 0.2 M to 0.5 M, most preferable between 0.3 to 0.4 M.Increase of the concentration of salt requires an increase in the concentration of organic solvent in order to achieve elution from the column within a suitable time. Physical instability involves conformational changes relative to the native structure, which includes loss of higher orderstructure, aggregation, fibrillation, precipitation or adsorptionto surfaces. Peptides such as insulin peptides, GLP-1 compounds and amylin compounds are known to be prone to instability due to fibrillation. Physicalstability of the formulations may be evaluated by conventionalmeans of e.g. visual inspection, nephelometry and Thioflavin Tassay after storage of the formulation at different temperatures for various time periods. Conformational stability can be evaluated by circular dichroism andNMR as described by e.g. Hudson and Andersen, Peptide Science, vol76 (4), pp. 298-308 (2004).

The biological activity of a polypeptide or a polypeptide derivative may be measured in an assay as known by a person skilled in the art as e.g. described in WO 2005/012347.

In one embodiment of the invention the pharmaceutical composition according to the invention is stable for more than 6 weeks of usage and for more than 3 years of storage.

In another embodiment of the invention the pharmaceutical composition according to the invention is stable for more than 4 weeks of usage and for more than 3 years of storage.

In a further embodiment of the invention the pharmaceutical composition according to the invention is stable for more than 4 weeks of usage and for more than two years of storage.

In an even further embodiment of the invention the pharmaceutical composition according to the invention is stable for more than 2 weeks of usage and for more than two years of storage.

In an even further embodiment of the invention the pharmaceutical composition according to the invention is stable for more than 1 weeks of usage and for more than one year of storage.

In one embodiment, the pharmaceutical composition according to the invention is used for the preparation of a medicament for the treatment or prevention of hyperglycemia, type 2 diabetes, impaired glucose tolerance, type 1 diabetes, obesity, hypertension, syndrome X, dyslipidemia, cognitive disorders, atherosclerosis, myocardial infarction, stroke, coronary heart disease and other cardiovascular disorders, inflammatory bowel syndrome, dyspepsia and gastric ulcers.

In another embodiment, the pharmaceutical composition according to the invention is used as a medicament for delaying or preventing disease progression in type 2 diabetes.

In another embodiment, the pharmaceutical composition according to the invention is used as a medicament for decreasing food intake, decreasing β-cell apoptosis, increasing β-cell function and β-cell mass, and/or for restoring glucose sensitivity to β-cells.

In one embodiment of the invention, the pharmaceutical composition according to the invention is for use as a medicament for the treatment or prevention of hyperglycemia, type 2 diabetes, impaired glucose tolerance, type 1 diabetes, obesity, hypertension, syndrome X, dyslipidemia, cognitive disorders, atherosclerosis, myocardial infarction, coronary heart disease and other cardiovascular disorders, stroke, inflammatory bowel syndrome, dyspepsia and gastric ulcers or for delaying or preventing disease progression in type 2 diabetes or for decreasing food intake, decreasing β-cell apoptosis, increasing β-cell function and β-cell mass, and/or for restoring glucose sensitivity to β-cells, is provided.

A further embodiment of the invention is, a method for the treatment or prevention of hyperglycemia, type 2 diabetes, impaired glucose tolerance, type 1 diabetes, obesity, hypertension, syndrome X, dyslipidemia, cognitive disorders, atherosclerosis, myocardial infarction, coronary heart disease and other cardiovascular disorders, stroke, inflammatory bowel syndrome, dyspepsia and gastric ulcers or for delaying or preventing disease progression in type 2 diabetes or for decreasing food intake, decreasing β-cell apoptosis, increasing β-cell function and β-cell mass, and/or for restoring glucose sensitivity to β-cells, the method comprising administering to a patient in need of such treatment an effective amount for such treatment of the pharmaceutical composition according to the invention.

The treatment with the pharmaceutical composition according to the invention may also be combined with treatment with a second or more pharmacologically active substances, e.g. selected from antidiabetic agents, antiobesity agents, appetite regulating agents, antihypertensive agents, agents for the treatment and/or prevention of complications resulting from or associated with diabetes and agents for the treatment and/or prevention of complications and disorders resulting from or associated with obesity. Examples of these pharmacologically active substances are: GLP-1 and GLP-1 derivatives and analogues, GLP-2 and GLP-2 derivatives and analogues, Exendin-4 and Exendin-4 derivatives and analogues, amylin and amylin derivatives and analogues, sulphonylureas, biguanides, meglitinides, glucosidase inhibitors, glucagon antagonists, DPP-IV (dipeptidyl peptidase-IV) inhibitors, inhibitors of hepatic enzymes involved in stimulation of gluconeogenesis and/or glycogenolysis, glucose uptake modulators, compounds modifying the lipid metabolism such as antihyperlipidemic agents as HMG CoA inhibitors (statins), compounds lowering food intake, RXR agonists and agents acting on the ATP-dependent potassium channel of the β-cells; Cholestyramine, colestipol, clofibrate, gemfibrozil, lovastatin, pravastatin, simvastatin, probucol, dextrothyroxine, neteglinide, repaglinide; β-blockers such as alprenolol, atenolol, timolol, pindolol, propranolol and metoprolol, ACE (angiotensin converting enzyme) inhibitors such as benazepril, captopril, enalapril, fosinopril, lisinopril, alatriopril, quinapril and ramipril, calcium channel blockers such as nifedipine, felodipine, nicardipine, isradipine, nimodipine, diltiazem and verapamil, and α-blockers such as doxazosin, urapidil, prazosin and terazosin; CART (cocaine amphetamine regulated transcript) agonists, NPY (neuropeptide Y) antagonists, MC4 (melanocortin 4) agonists, orexin antagonists, TNF (tumor necrosis factor) agonists, CRF (corticotropin releasing factor) agonists, CRF BP (corticotropin releasing factor binding protein) antagonists, urocortin agonists, β3 agonists, MSH (melanocyte-stimulating hormone) agonists, MCH (melanocyte-concentrating hormone) antagonists, CCK (cholecystokinin) agonists, serotonin re-uptake inhibitors, serotonin and noradrenaline re-uptake inhibitors, mixed serotonin and noradrenergic compounds, 5HT (serotonin) agonists, bombesin agonists, galanin antagonists, growth hormone, growth hormone releasing compounds, TRH (thyreotropin releasing hormone) agonists, UCP 2 or 3 (uncoupling protein 2 or 3) modulators, leptin agonists, DA agonists

(bromocriptin, doprexin), lipase/amylase inhibitors, RXR (retinoid X receptor) modulators, TR β agonists; histamine H3 antagonists, gastrin and gastrin analogues and derivatives.

It should be understood that any suitable combination of therapeutically active polypeptides in the pharmaceutical composition according to the invention and optionally one or more further pharmacologically active substances are considered to be within the scope of the present invention.

### FURTHER EMBODIMENTS ACCORDING TO THE INVENTION

1. A solid or semi-solid pharmaceutical composition comprising a water soluble polypeptide (a), at least one polar organic solvent (b) for the polypeptide, at least one surfactant (c), at least one lipophilic component (d), and optionally at least one solid hydrophilic component (e),wherein said pharmaceutical composition is spontaneously dispersible.
2. The pharmaceutical composition according to embodiment 1, which comprises less than 10% w/w water.
3. The pharmaceutical composition according to any one of embodiments 1-2, which comprises less than 5% w/w water.
4. The pharmaceutical composition according to any one of embodiments 1-3, which comprises less than 2% w/w water.
5. The pharmaceutical composition according to any one of embodiments 1-4, wherein the polar organic solvent is selected from the group consisting of polyols.
6. The pharmaceutical composition according to any one of embodiments 1-5, wherein the polarorganic solvent is selected from the group consisting of diols and triols.
7. The pharmaceutical composition according to any one of embodiments 1-6, wherein the polarorganic solvent is selected from the group consisting of propylene glycol, glycerol and mixtures thereof.
8. The pharmaceutical composition according to embodiment 7, wherein the polarorganic solvent is propylene glycol.
9. The pharmaceutical composition according to embodiment 7, wherein the polarorganic solvent is glycerol.
10.The pharmaceutical composition according any one of embodiments 1-9, wherein the polypeptide is selected from the group consisting of insulin peptides, insulinotropic compounds, amylin, amylin analogues, amylin derivatives, α-MSH , α-MSH analogues, α-MSH derivatives and/or any combination thereof.
11. The pharmaceutical composition according to any one of embodiments 1-10, wherein the insulintropic compound is an insulinotropic peptide or analogue.
12.The pharmaceutical composition according any one of embodiments 1-11, wherein the insulintropic compound is an insulinotropic peptide which is a DPP-IV protected peptide.
13.The pharmaceutical composition according to any one of embodiments 1-12, wherein said insulinotropic peptide comprises a lipophilic substituent selected from the group consisting of CH₃(CH₂)ₙCO- wherein n is 4 to 38, and HOOC(CH₂)ₘCO- wherein m is from 4 to 38.
14.The pharmaceutical composition according any one of embodiments 1-13, wherein said insulinotropic peptide is acylated GLP-1 or an acylated GLP-1 analogue.
15.The pharmaceutical composition according to embodiment 14, wherein said GLP-1 analogue is selected from the group consisting of Arg³⁴-GLP-1(7-37), Gly⁸-GLP-1(7-36)-amide, Gly⁸-GLP-1(7-37), Val⁸-GLP-1(7-36)-amide, Val⁸-GLP-1(7-37), Aib⁸-GLP-1(7-36)-amide, Aib⁸-GLP-1(7-37), Val⁸Asp²²-GLP-1(7-36)-amide, Val⁸Asp²²-GLP-1(7-37) , Val⁸Glu²²-GLP-1(7-36)-amide , Val⁸Glu²²-GLP-1(7-37), Val⁸Lys²²-GLP-1(7-36)-amide, Val⁸Lys²²-GLP-1(7-37), Val⁸Arg²²-GLP-1(7-36)-amide, Val⁸Arg²²-GLP-1(7-37), Val⁸His²²-GLP-1(7-36)-amide, Val⁸His²²-GLP-1(7-37), Val⁸Trp¹⁹Glu²²-GLP-1(7-37), Val⁸Glu²²Val²⁵-GLP-1(7-37), Val⁸Tyr¹⁶Glu²²-GLP-1(7-37), Val⁸Trp¹⁶Glu²²-GLP-1(7-37), Val⁸Leu¹⁶Glu²²-GLP-1(7-37), Val⁸Tyr¹⁸Glu²²-GLP-1(7-37), Val⁸Glu²²His³⁷-GLP-1(7-37), Val⁸Glu²²Ile³³-GLP-1(7-37), Val⁸Trp¹⁶Glu²²Val²⁵Ile³³-GLP-1(7-37), Val⁸Trp¹⁶Glu²²Ile³³-GLP-1(7-37), Val⁸Glu²²Val²⁵Ile³³-GLP-1(7-37), Val⁸Trp¹⁶Glu²²Val²⁵-GLP-1(7-37), and analogues thereof.
16.The pharmaceutical composition according to embodiment 15, wherein said insulinotropic peptide is Arg34, Lys26(Nε-(γ-Glu(Nα-hexadecanoyl)))-GLP-1(7-37).
17.The pharmaceutical composition according to embodiment 11, wherein said insulinotropic peptide is exendin-4 or ZP-10, i.e. HGEGTFTSDLSKQMEEEAVRLFIEWLKNGGPSSGAPPSKKKKKK-NH2.
18.The pharmaceutical composition according embodiment 11, wherein said insulinotropic peptide is acylated exendin-4 or an acylated exendin-4 analogue.
19.The pharmaceutical composition according to embodiment 11, wherein said insulinotropic peptide is [N-epsilon(17-carboxyheptadecanoic acid)Lys20 exendin-4(1-39)-amide [SEQ ID No 3] 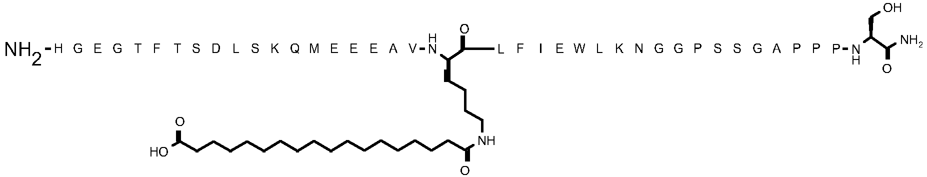 or
   N-epsilon32-(17-carboxy-heptadecanoyl)[Lys32]exendin-4(1-39)amide [SEQ ID No 4] 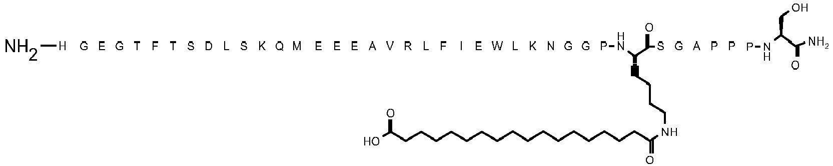
20.The pharmaceutical composition according to any one of embodiments 1-11, wherein the insulin peptide is an insulin analogue.
21.The pharmaceutical composition according to any one of embodiments 1-11, wherein the insulin peptide is an insulin analogue selected from the group consisting of AspB28 human insulin; LysB28ProB29 human insulin; LysB3GluB29 human insulin and A14GluB25HisdesB30 human insulin.
22.The pharmaceutical composition according to any one of the embodiments 1-21, wherein the polar organic solvent is a polyol.
23.The pharmaceutical composition according to any one of the embodiments 1-22, wherein the polar organic solvent is a mixture of glycerol and propylene glycol.
24.The pharmaceutical composition according to any one of the embodiments 1-23, wherein the polar organic solvent is propylene glycol.
25.The pharmaceutical composition according to any one of the embodiments 1-24, wherein the surfactant is a non ionic surfactant.
26.The pharmaceutical composition according to any one of the embodiments 1-25, wherein the surfactant is a polyoxyethylene containing surfactant.
27.The pharmaceutical composition according to any one of the embodiments 1-26, wherein the surfactant is a solid surfactant selected from the group consisting of a poloxamer and a mixture of poloxamers such as Pluronic F-127 or Pluronic F-68.
28.The pharmaceutical composition according to any one of the embodiments 1-27, wherein the lipophilic componentis a phospholipid.
29.The pharmaceutical composition according to any one of the embodiments 1-28, wherein the lipophilic component is a mono-di-triglyceride.
30.The pharmaceutical composition according to any one of the embodiments 1-29, wherein the lipophilic component is a mono-di-glyceride.
31.The pharmaceutical composition according to any one of the embodiments 1-30, which is solid.
32.The pharmaceutical composition according to any one of the embodiments 1-31, which is semi-solid.
33.The pharmaceutical composition according to any one of the embodiments 1-32, which is solid at room temperature and liquid at body temperature.
34.The pharmaceutical composition according to any one of the embodiments 1-33, wherein (c) is solid or semi-solid.
35.The pharmaceutical composition according to any one of the embodiments 1-34, wherein (d) is solid or semi-solid.
36.The pharmaceutical composition according to any one of the embodiments 1-33, which comprises a solid hydrophilic component (e).
37.The pharmaceutical composition according to any one of the embodiments 1-36 for use as a medicament in the treatment of hyperglycemia.
38. The pharmaceutical composition according to any one of the embodiments 1-36 for use as a medicament.
39.The pharmaceutical composition according to any one of the embodiments 1-36 for use as a medicament in the treatment of obesity.
40.The pharmaceutical composition according to any one of the embodiments 1-36 for use as a medicamentin the treatment of binge eating or bulimia.
41.A method for treatment of hyperglycemia comprising oral administration of an effective amount of the pharmaceutical composition as defined in any of the embodiments 1-36.
42.A method for treatment of obesity comprising oral administration of an effective amount of the pharmaceutical composition as defined in any of the embodiments 1-36.
43.A method for treatment of binge eating or bulimia comprising oral administration of an effective amount of the pharmaceutical composition as defined in any of the embodiments 1-36.

### The following is a list of aspects further describing the invention:

1a. A solid or semi-solid pharmaceutical composition comprising a water soluble polypeptide (a), at least one polar organic solvent (b) for the polypeptide, at least one surfactant (c), at least one lipophilic component (d), and optionally at least one solid hydrophilic component (e),wherein said pharmaceutical composition is spontaneously dispersible.
2a. The pharmaceutical composition according to aspect 1a, which comprises less than 10% w/w water.
3a. The pharmaceutical composition according to any one of aspects 1a-2a, wherein the organic solvent is selected from the group consisting of polyols.
4a. The pharmaceutical composition according to any one of aspects 1a-3a, wherein the organic solvent is selected from the group consisting of propylene glycol, glycerol and mixtures thereof.
5a. The pharmaceutical composition according to aspect 4a, wherein the organic solvent is propylene glycol.
6a. The pharmaceutical composition according any one of aspects 1a-5a, wherein the polypeptide is selected from the group consisting of insulin peptides, insulinotropic compounds, amylin, amylin analogues, amylin derivatives, α-MSH , α-MSH analogues, α-MSH derivatives and/or any combination thereof.
7a. The pharmaceutical composition according to any one of aspects 1a-6a, wherein the insulintropic compound is an insulinotropic peptide.
8a. The pharmaceutical composition according to any one of aspects 1a-7a, wherein the insulin peptide is an insulin analogue.
9a. The pharmaceutical composition according to any one of aspects 1a-8a, wherein the insulin peptide is an insulin analogue selected from the group consisting of AspB28 human insulin; LysB28ProB29 human insulin; LysB3GluB29 human insulin and A14GluB25HisdesB30 human insulin.
10a. The pharmaceutical composition according to any one of the aspects 1a-9a, wherein the surfactant is a non-ionic surfactant.
11a. The pharmaceutical composition according to any one of the aspects 1a-10a, wherein the surfactant is a solid surfactant selected from the group consisting of a poloxamer and a mixture of poloxamers such as Pluronic F-127 or Pluronic F-68.
12a. The pharmaceutical composition according to any one of the aspects 1a-11a, wherein the lipophilic component is a mono-di-glyceride.
13a. The pharmaceutical composition according to any one of the aspects 1a-12a, which comprises a solid hydrophilic component (e).
14a. The pharmaceutical composition according to any one of the aspects 1a-13a for use as a medicament in the treatment of hyperglycemia.
15a. A method for treatment of hyperglycemia comprising oral administration of an effective amount of the pharmaceutical composition as defined in any of the aspects 1a-14a.

The term "about" as used herein means in reasonable vicinity of the stated numerical value, such as plus or minus 10%.

All references, including publications, patent applications and patents, cited herein are hereby incorporated by reference to the same extent as if each reference was individually and specifically indicated to be incorporated by reference and was set forth in its entirety herein.

All headings and sub-headings are used herein for convenience only and should not be construed as limiting the invention in any way,

Any combination of the above-described elements in all possible variations thereof is encompassed by the invention unless otherwise indicated herein or otherwise clearly con-tradicted by context.

The terms "a" and "an" and "the" and similar referents as used in the context of describing the invention are to be construed to cover both the singular and the plural, unless otherwise indicated herein or clearly contradicted by context.

Recitation of ranges of values herein are merely intended to serve as a shorthand method of referring individually to each separate value falling within the range, unless otherwise indicated herein, and each separate value is incorporated into the specification as if it were individually recited herein. Unless otherwise stated, all exact values provided herein are representative of corresponding approximate values (*e.g*., all exact exemplary values provided with respect to a particular factor or measurement can be considered to also pro-vide a corresponding approximate measurement, modified by "about," where appropriate).

All methods described herein can be performed in any suitable order unless otherwise indicated herein or otherwise clearly contradicted by context.

The use of any and all examples, or exemplary language (*e.g*., "such as") provided herein, is intended merely to better illuminate the invention and does not pose a limitation on the scope of the invention unless otherwise indicated. No language in the specification should be construed as indicating any element is essential to the practice of the invention unless as much is explicitly stated.

The citation and incorporation of patent documents herein is done for convenience only and does not reflect any view of the validity, patentability and/or enforceability of such patent documents,

The description herein of any aspect or embodiment of the invention using terms such as "comprising", "having", "including" or "containing" with reference to an element or elements is intended to provide support for a similar aspect or embodiment of the invention that "consists of", "consists essentially of", or "substantially comprises" that particular element or elements, unless otherwise stated or clearly contradicted by context (*e.g*., a formulation described herein as comprising a particular element should be understood as also describing a formulation consisting of that element, unless otherwise stated or clearly contradicted by context).

This invention includes all modifications and equivalents of the subject matter recited in the aspects or claims presented herein to the maximum extent permitted by applicable law.

The present invention is further illustrated in the following representative methods and examples which are, however, not intended to limit the scope of the invention in any way.

The features disclosed in the foregoing description and in the following examples may, both separately and in any combination thereof, be material for realising the invention in diverse forms thereof.

### EXAMPLES

### Example 1Preparation and measurement of droplets

A composition comprising propylene glycol, Capmul MCM, poloxamer 407 and PEG 3350 wasprepared. 600 mg Capmul MCM, 200 mg poloxamer 407 and 200 mg PEG 3350 weremelted at 58 °C and then mixed with 1000 mg propylene glycol (37 °C). After dilution in aqueous medium the droplet sizewasmeasured with a Zetasizer Nano ZS at 37 °C.

| Dilution in MilliQ water | Polydispersity Index | Average diameter [nm] |
|---|---|---|
| 1:10 | 0.228 | 278 |
| 1:50 | 0.22 | 207 |
| 1:100 | 0.183 | 208 |

### Example 2Oral administration of a pharmaceutical composition comprising insulin aspart

Insulin Aspart was dissolved in propylene glycol at room temperature (RT), mixed with the other components (pre-melted at 58 °C) to give a clear phase, filled into enteric coated HPMC capsules and stored in the fridge in order to solidify.

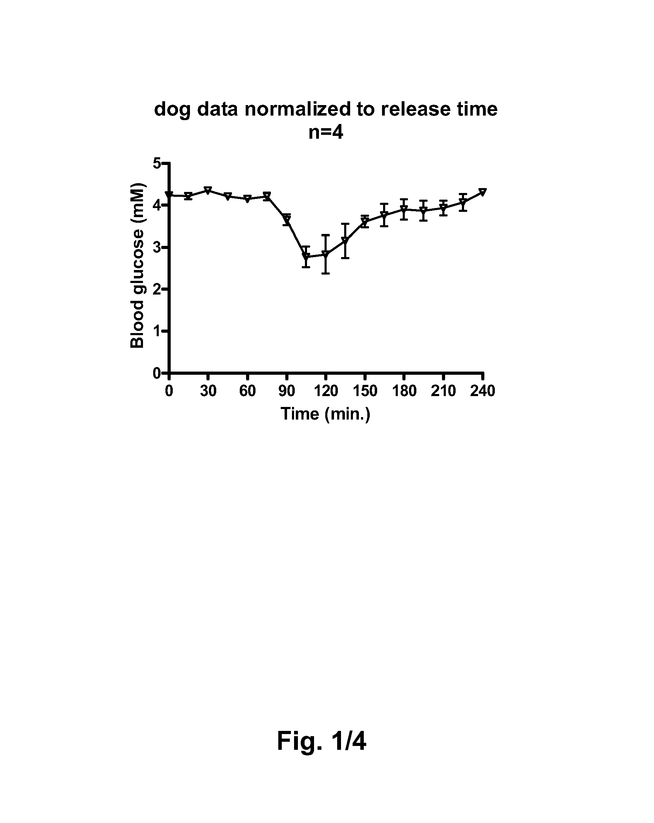

4 overnight fasted non diabetic dogs (body weight (BW) 17 kg) were dosed orally with an enteric coated capsulecontaining 69 mg insulin aspart, 447 mg propylene glycol, 200 mg Capmul MCM, 66.7 mg Pluronic F127 and 66.7 mg PEG 3350.Reduction in blood glucose levels is shown in Figure 1.

### Example 3Oral administration of a pharmaceutical composition comprising the insulin analogue A14GluB25HisdesB30 human insulin

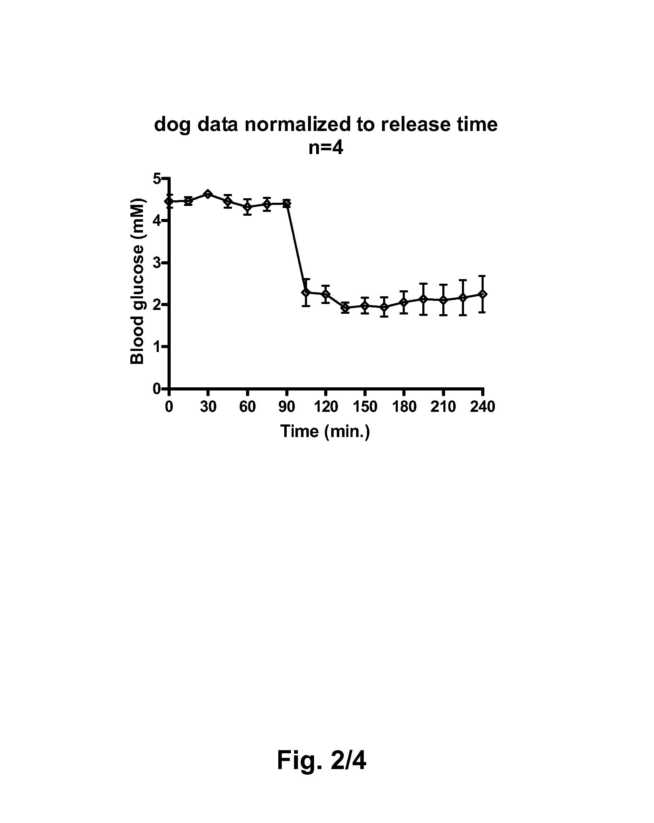

The insulin analoguewas dissolved in propylene glycol at RT, mixed with the other components (pre-melted at 58 °C) to give a clear phase, filled into enteric coated HPMC capsules and stored in the fridge in order to solidify. 4 overnight fasted non diabetic dogs (BW 17 kg) were dosed orally with an enteric coated capsule containing 46 mg A14GluB25HisdesB30 human insulin,431 mg propylene glycol, 223.8 mg Capmul MCM, 74.6 mg Pluronic F127 and 74.6 mg PEG 3350.Reduction in blood glucose levels is shown in Figure 2.

### Example 4Reduction of the blood glucose level after per-oral dosage (via gavage) of insulin aspart or vehicle controls to non-diabetic SPRD rats

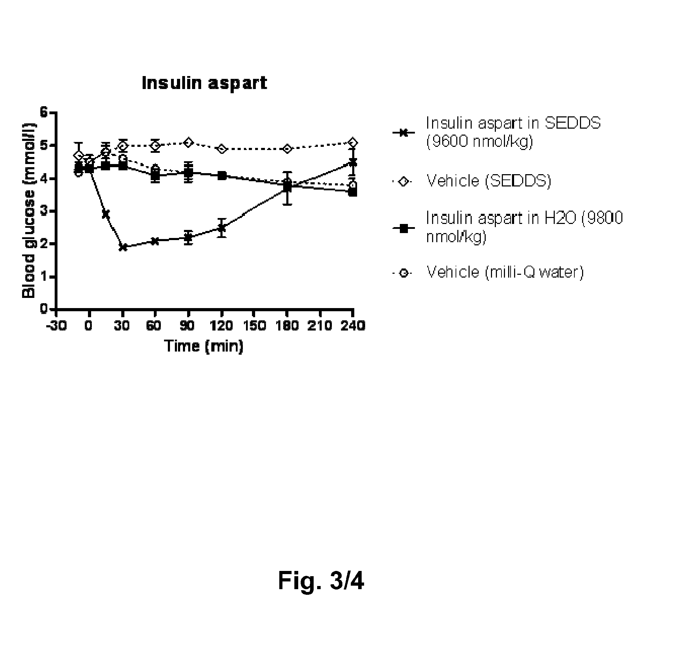

The reduction in blood glucose in non-diabetic rats was examined after:
1) insulin aspart was dosed orally in a concentration of 9600 nmol/kg in aspontaneously dispersible preconcentrate drug delivery system (SEDDS) comprising insulin aspart dissolved in propylene glycol (62.5% of the total composition) and mixed with melted Capmul MCM C10 (31.25% of the total composition) and poloxamer 407 (6.25% of the total composition),
2) Insulin aspart was dosed orally in a concentration of 9800 nmol/kg in H₂O, and
3) Vehicle SEDDS containing62.5% propylene glycol, 31.25% Capmul MCM C10 and 6.25% poloxamer 407was dosed orally. SEDDS hadbeen melted at 35°C before dosing.
(mean ± SEM, n=5-6, dosage volume = 4 ml/kg). The resulting reduction in blood glucose levels is shown in Figure 3.

### Example 5Reduction of the blood glucose level after per-oral dosage (via gavage) of A14GluB25HisdesB30 human insulin or vehicle controls to non-diabetic SPRD rats

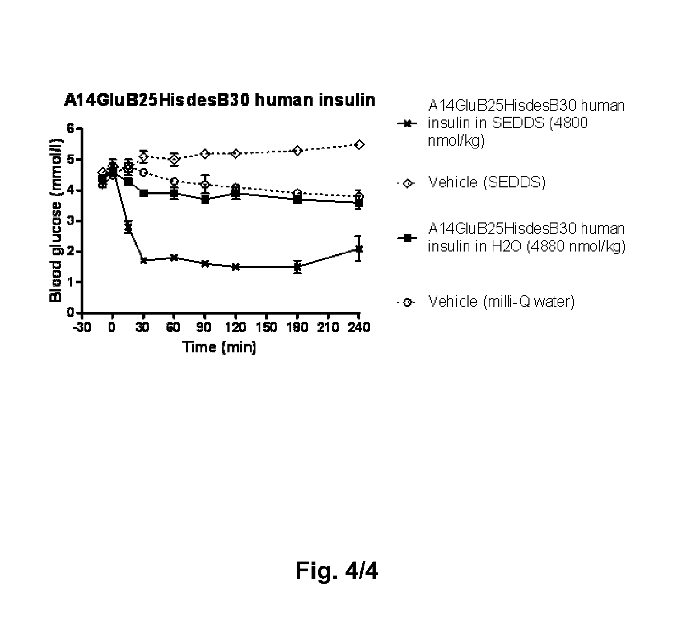

The reduction in blood glucose in non-diabetic rats was examined after:
1) A14GluB25HisdesB30 human insulin was dosed orally in a concentration of 4800 nmol/kg in a spontaneously dispersible preconcentratedrug delivery system (SEDDS) comprising A14GluB25HisdesB30 human insulin dissolved in propylene glycol (62.5% of the total composition) and mixed with melted Capmul MCM C10 (31.25% of the total composition) and poloxamer 407 (6.25% of the total composition).
2) A14GluB25HisdesB30 human insulin was dosed orally in a concentration of 4880 nmol/kg in H₂O.
3) Vehicle SEDDS containing62.5% propylene glycol, 31.25% Capmul MCM C10 and 6.25% poloxamer 407 was dosed orally. SEDDS hadbeen melted at 35°C before dosing.
(mean ± SEM, n=5-6, dosage volume = 4 ml/kg).The resulting reduction in blood glucose levels is shown in Figure 4.

## Claims

1. A solid or semi-solid pharmaceutical composition comprising
a. A spontaneously dispersible preconcentrate comprising at least one polar organic solvent, at least one surfactant, at least one lipophilic component, and optionally at least one solid hydrophilic component, -and
b. a water soluble polypeptide

2. The pharmaceutical composition according to claim 1, which comprises less than 10% w/w water.

3. The pharmaceutical composition according to any one of claims 1-2, wherein the organic solvent is selected from the group consisting of polyols.

4. The pharmaceutical composition according to any one of claims 1-3, wherein the organic solvent is selected from the group consisting of propylene glycol, glycerol and mixtures thereof.

5. The pharmaceutical composition according to claim 4, wherein the organic solvent is propylene glycol.

6. The pharmaceutical composition according any one of claims 1-5, wherein the polypeptide is selected from the group consisting of insulin peptides, insulinotropic compounds, amylin, amylin analogues, amylin derivatives, α-MSH , α-MSH analogues, α-MSH derivatives and/or any combination thereof.

7. The pharmaceutical composition according to any one of claims 1-6, wherein the insulintropic compound is an insulinotropic peptide.

8. The pharmaceutical composition according to any one of claims 1-7, wherein the insulin peptide is an insulin analogue.

9. The pharmaceutical composition according to any one of claims 1-8, wherein the insulin peptide is an insulin analogue selected from the group consisting of AspB28 human insulin; LysB28ProB29 human insulin; LysB3GluB29 human insulin and A14GluB25HisdesB30 human insulin.

10. The pharmaceutical composition according to any one of the claims 1-9, wherein the surfactant is a non-ionic surfactant.

11. The pharmaceutical composition according to any one of the claims 1-10, wherein the surfactant is a solid surfactant selected from the group consisting of a poloxamer and a mixture of poloxamers.

12. The pharmaceutical composition according to any one of the claims 1-11, wherein the lipophilic component is one or more selected from the group consisting of mono- and diglycerides.

13. The pharmaceutical composition according to any one of the claims 1-12, which comprises a solid hydrophilic component.

14. The pharmaceutical composition according to any one of the claims 1-13 for use as a medicament in the treatment of hyperglycemia.

15. A method for treatment of hyperglycemia comprising oral administration of an effective amount of the pharmaceutical composition as defined in any of the claims 1-14.


SEQUENCE LISTING

<110>  Novo Nordisk A/S

<120>  SPONTANEOUSLY DISPERSIBLE PRECONCENTRATES INCLUDING

A PEPTIDE DRUG IN A SOLID OR SEMISOLID CARRIER

<130>  7713.504-WO

<160>  4

<170>  PatentIn version 3.4

<210>  1
<211>  31
<212>  PRT
<213>  homo sapiens

<400>  1

His Ala Glu Gly Thr Phe Thr Ser Asp Val Ser Ser Tyr Leu Glu Gly
1               5                   10                  15

Gln Ala Ala Lys Glu Phe Ile Ala Trp Leu Val Lys Gly Arg Gly
            20                  25                  30

<210>  2
<211>  40
<212>  PRT
<213>  Heloderma suspectum

<220>
<221>  MISC_FEATURE
<222>  (40)..(40)
<223>  Xaa is NH2

<400>  2

His Gly Glu Gly Thr Phe Thr Ser Asp Leu Ser Lys Gln Met Glu Glu
1               5                   10                  15

Glu Ala Val Arg Leu Phe Ile Glu Trp Leu Lys Asn Gly Gly Pro Ser
            20                  25                  30

Ser Gly Ala Pro Pro Pro Ser Xaa
        35                  40

<210>  3
<211>  40
<212>  PRT
<213>  Artificial

<220>
<223>  Derivative of Exendin-4

<220>
<221>  MISC_FEATURE

<222>  (20)..(20)
<223>  Xaa is N-epsilon(17-carboxyheptadecanoic acid)Lys

<220>
<221>  MISC_FEATURE
<222>  (40)..(40)
<223>  Xaa is NH2

<400>  3

His Gly Glu Gly Thr Phe Thr Ser Asp Leu Ser Lys Gln Met Glu Glu
1               5                   10                  15

Glu Ala Val Xaa Leu Phe Ile Glu Trp Leu Lys Asn Gly Gly Pro Ser
            20                  25                  30

Ser Gly Ala Pro Pro Pro Ser Xaa
        35                  40

<210>  4
<211>  40
<212>  PRT
<213>  Artificial

<220>
<223>  Derivative of Exendin-4

<220>
<221>  MISC_FEATURE
<222>  (32)..(32)
<223>  Xaa is N-epsilon-(17-carboxy-heptadecanoyl)Lys

<220>
<221>  MISC_FEATURE
<222>  (40)..(40)
<223>  Xaa is NH2

<400>  4

His Gly Glu Gly Thr Phe Thr Ser Asp Leu Ser Lys Gln Met Glu Glu
1               5                   10                  15

Glu Ala Val Arg Leu Phe Ile Glu Trp Leu Lys Asn Gly Gly Pro Xaa
            20                  25                  30

Ser Gly Ala Pro Pro Pro Ser Xaa
        35                  40